(19) Europäisches Patentamt
European Patent Office
Office européen des brevets

(11) EP 0 986 381 B1

(12) EUROPEAN PATENT SPECIFICATION

(45) Date of publication and mention of the grant of the patent:
**16.06.2004 Bulletin 2004/25**

(21) Application number: **98918524.4**

(22) Date of filing: **15.04.1998**

(51) Int Cl.[7]: **A61K 31/335**, C07D 303/06, A61P 31/12, A61P 31/18, A61P 31/22, A61P 35/00, A61P 37/06

(86) International application number:
**PCT/US1998/008013**

(87) International publication number:
**WO 1998/046222 (22.10.1998 Gazette 1998/42)**

(54) **USE OF DITERPENES LIKE HYPOESTOXIDES FOR THE MANUFACTURE OF A MEDICAMENT FOR IMMUNOSUPPRESSION, ANTICANCER AND ANTIVIRAL TREATMENT**

VERWENDUNG VON DITERPENEN WIE HYPOESTOXIDEN ZUR HERSTELLUNG EINES MEDIKAMENTES ZUR IMMUNSUPPRESSION, KREBS- UND ANTIVIRALEN THERAPIE

UTILISATION DES DITERPENES COMME HYPOESTOXIDES POUR LA PRODUCTION D'UN MéDICAMENT POUR LE TRAITEMENT IMMUNOSUPPRESSIV, ANTICANCEREUX ET ANTIVIRAL

(84) Designated Contracting States:
**DE ES FR GB IT**

(30) Priority: **15.04.1997 US 843401**
**14.01.1998 US 6946**
**15.01.1998 US 7308**

(43) Date of publication of application:
**22.03.2000 Bulletin 2000/12**

(73) Proprietor: **Immune Modulation, Inc.**
**Bloomington, CA 92316 (US)**

(72) Inventors:
- **OJO-AMAIZE, Emmanuel, A.**
  **Glendora, CA 91740 (US)**
- **OKOGUN, Joseph, I.**
  **New Rochelle, NY 10801 (US)**
- **COTTAM, Howard, B.**
  **Fallbrook, CA 92028 (US)**
- **KAKKANAIAH, Vellalore, N.**
  **Los Angeles, CA 90034 (US)**

(74) Representative: **Atkinson, Peter Birch et al**
**MARKS & CLERK,**
**Sussex House,**
**83-85 Mosley Street**
**Manchester M2 3LG (GB)**

(56) References cited:
- **ADESOMOJU A ET AL: "HYPOESTOXIDE, A NEW DITERPENE FROM HYPOESTES ROSEA (ACANTHACEAE)" HETEROCYCLES, XX, XX, vol. 20, no. 11, 1983, pages 2125-2128, XP000980187 ISSN: 0385-5414**
- **SUGANO et al., "Structure-Activity Relationships of Phomactin Derivatives as Platelet Activating Factor Antagonists", J. MED. CHEM., 1996, Vol. 39, No. 26, pages 5281-5284, XP002914036**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 986 381 B1

## Description

## BACKGROUND OF THE INVENTION

### 1. Field of the Invention

This invention relates to compounds as used for immunosuppressive agents and their use in transplantation therapy. In particular, the invention is directed to the use of diterpene compounds, in particular hypoestoxides, derivatives and agonists thereof for immunosuppressing, inflammation, graft rejection, graft-versus-host-disease (GVHD), skin diseases, and T-cell-mediated autoimmune diseases such as arthritis, systemic lupus erythematosus, thyroiditis, multiple sclerosis, glomerulonephritis, diabetes, and allergy. The invention further relates to use of the compounds for inhibiting growth of cancer cells, and for inhibiting the growth or lentiviruses or *Herpetoviridae* viruses. In addition, the invention relates to the use of the compounds for inhibiting growth of cancer cells.

### 2. Background Art

Immunosuppressive Agents. Many human diseases are characterized by excessive or inappropriate immune responses. In transplantation, the immune system attacks major histocompatibility complex (MHC)-disparate donor tissue leading to graft rejection. In allergy or inflammation, the immune system is hyperresponsive to otherwise harmless environmental antigens. In autoimmune diseases, the immune system attacks normal self-tissues. In immunodeficiency diseases such as HIV infection and AIDS, an infectious agent attacks the host's immune system. It is now recognized that immunosuppressive therapy is appropriate for treating each of these disorders (Blood Reviews 1995;9:117-133). The development of naturally occurring secondary metabolites of cyclosporin A (CsA), FK506 and rapamycin as immunosuppressants has revolutionized organ transplantation through their use in the prevention of graft rejection (TIBS 1993;18:334-338) and GVHD (Ann Hematol 1995;71:301-306). There are, however, serious side effects associated with use of these drugs, such as nephrotoxicity, neurotoxicity, hepatotoxicity, endocrine complications and bone effects (New Engl J Med (1989)321:1725-1738; Kahan BD et al. Surgery (1985) 97:125). There is, therefore, a clinical need to provide new compounds for effective immunosuppression without these side effects.

Anticancer Agents. The strongest impetus for the many decades of research in tumor biology has been the hope that effective therapeutic agents might be found cytotoxic against tumor cells that could be used for prevention and therapy of cancer.

Malignant melanoma continues to increase in incidence. Currently, malignant melanoma is the fifth most frequently occurring cancer in the U.S. While early melanoma is highly curable by surgical means, the prognosis of patients with more advanced lesions and/or metastatic disease remains poor (Bezwoda, WR; Cancer Treat Rev 1997; 23(1):17-34). Conventional chemotherapy with single-agent activity has a low frequency and short duration of response (Cancer Treat Rev 1997; 23(1):17-34); Mayo Clin Proc 1997;72:367-371).

Metastatic renal cell carcinoma (RCC) is highly resistant to the many systemic therapies that have been extensively investigated (Motzer RJ et al. Current Problems in Cancer, 1997;21(4):185-232). RCC occurs nearly twice as often in men as in women. Increased availability of improved and highly sophisticated diagnostic methods such as ultrasonography and computed tomography (CT) scanning (Franklin JR et al. Seminars in Urologic Oncology 1996;14 (2):208-215) has revealed a steadily rising incidence of RCC. Cervical carcinoma is the most common malignancy among women in developing countries (Morrow CP et al. J Cell Biochem 1995; suppl 23:61-70) and the third most common malignancy of the female genital tract in the United States (Miller BA et al. National Cancer Institute; NIH publication 93-2789, 1993).

All of the subsequent technological advances, including high dose rate therapy, treatment planning, new isotopes, new imaging techniques, and new machines, have had no clinically measurable effect on the curability of cervical cancer (Morrow CP et al. J Cell Biochem 1995; suppl 23:61-70).

Because neurotoxicity and chemotherapeutic drug resistance remain some of the major clinical problems associated with failure in the therapy of human cancer (Anticancer Drugs 1995;6(3):369-383; Curr Opin Oncol 1997; 9(1):79-87), there is a need for study of new agents with fewer side effects and maximum efficacy.

Antiviral Agents. The interactions between viruses and the host immune system are not only complex and fascinating but also critical in determining the outcome of infection and strategies for its prevention. The goal of antiviral chemotherapy is to inhibit replication of the viral genome without affecting the DNA of the cell.

Of the large number of agents under development for the treatment of herpes virus infections [Herpes Simplex Virus types 1 and 2 (HSV-1 and HSV-2), Varicella Zoster Virus (VZV), cytomegalovirus (CMV), Epstein-Barr virus (EBV)], only ten have apparently reached clinical development (Alrabiah FA and Sacks, SL; Drugs 1996:52(1):17-32). Although aciclovir is the treatment of choice in herpes simplex encephalitis (HSV-1), mortality and morbidity remain problematic (Sköldenberg B; Scand. J. Infect Dis Suppl 100:8-13, 1996). The same can be said about interferon-alpha

(IFN-α) and interferon-beta (IFN-β). Although both IFNs have considerable antiviral and immunomodulatory effects, their success as antiviral agents in humans has been hindered by their dose-limiting side effects (Balkwill, FR. Interferons; Lancet 1989;1:1060-1063). HSV-2 is the most common infective cause of genital ulceration in developed countries. Currently, 1 in 5 (20%) teenage adults in the United States is infected with genital herpes. A range of antiviral agents has become available since the early 1980s which can reduce disease severity, but HSV infection is life-long and, once established, there is no treatment which will eliminate it (Brugha, R. et al. Int. J. Epidemiol 1997;26:698-709). Therefore, there is a tremendous need to develop new approaches and agents to eliminate HSV infection.

The number of Human Immunodeficiency Virus type 1 (HIV-1)-infected individuals is currently estimated at 1-2 million in the United States, with a worldwide incidence of approximately 20 million. By the year 2000, it is estimated that more than 3 million Americans will be infected with HIV-1.

Until recently, treatment of HIV-1 infection was limited to the use of nucleoside inhibitors ofthe viral enzyme reverse transcriptase (RTI). While these agents initially offered promise, they have only modest antiviral activity and the benefits of treatment are limited by the emergence of drug resistance and dose-limiting toxic effects (McDonald CK et al. Arch Intern Med 1997;157(9):951-959). Although treatment of HIV with nucleoside analog RTIs and protease inhibitors forms the backbone of anti-HIV therapy, non-nucleoside RTIs, immune modulators, and new entries in existing classes of pharmacologic agents hold promise for the future (Hartman AF, Prim Care 1997;24(3):531-560). Because combination therapy with two, three, or more agents has become the standard of care, additive toxicities have become a major problem.

DISCLOSURE OF THE INVENTION

Immunosuppression. Applicants' invention rests on their finding that a select group of hypoestoxide analogs possess unexpected effectiveness as immunosuppressive agents.

Accordingly, one aspect of the invention is directed to compounds of the formula

IV

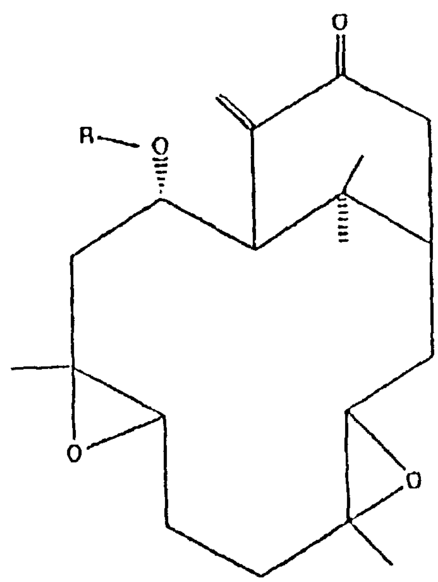

where:

R is

(i) H, $PO_3^{=}$, alkyl of 1 to 12 carbon atoms substituted or unsubstituted, straight chain or branched, 0 to 6 double bonds, $(CH_2)_n$morpholine where n=1-4, morpholinomethylphenyl, orthoaminophenyl, orthohydroxyphenyl, $(CH_2)_nCOOR_2$ where n =1-4

where $R_2$ is H, an alkalai metal salt, an alkaline earth metal salt, $NH_4^+$, $N^+(R_3)_4$ where $R_3$ is independently selected from the group consisting of H and alkyl of 1 to 4 carbon atoms, or

(ii) $COR_1$ wherein $R_1$ is selected from the group consisting of H, $(CH_2)_nCH_3$, where n=1-6, $(CH_2)_nCOOR_2$

where n=1-4 and $R_2$ is previously defined, $(CH_2)_nN^+(R_3)_4$ wherein n=1-4, and $(CH_2)_nSO_3^-$ where n=1-4,

and pharmaceutically acceptable salts thereof.

A related aspect is directed to pharmaceutical compositions comprising a therapeutically effective immunosuppressant amount of the compounds of formula IV.

Another aspect of the invention is directed to methods for inducing immunosuppression in animals which need immunosuppressive treatment. The method comprises administering to animals pharmaceutical compositions comprising a therapeutically effective immunosuppresant amount of hypoestoxide (herein designated JO-4 and illustrated in formula I) or one or more of the compounds of formula IV.

IV

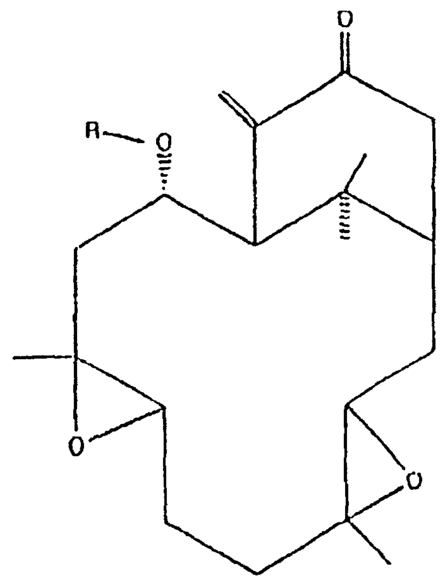

Anticancer Agents. Applicants' invention rests on their finding that a select group of hypoestoxide analogs possess unexpected effectiveness as anticancer agents. In particular, the present invention comprises a method for inhibiting the growth of cancer cells or cancerous tumors. The method comprises administering to a subject in need of anticancer or anticancer therapy a pharmaceutical composition comprising a therapeutically effective amount of a compound of the formula IV.

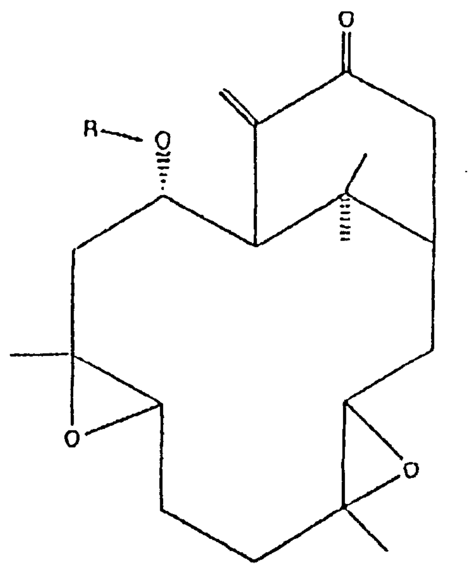

IV

where:

R is

(i) H, $PO_3^=$, alkyl of 1 to 12 carbon atoms substituted or unsubstituted, straight chain or branched, 0 to 6 double bonds, $(CH_2)_n$morpholine where n=1-4, morpholinomethylphenyl, orthoaminophenyl, orthohydroxyphenyl, $(CH_2)_nCOOR_2$ where n =1-4

where $R_2$ is H, an alkali metal salt, an alkaline earth metal salt, $NH_4^+$, $N^+(R_3)_4$ where $R_3$ is independently selected from the group consisting of H and alkyl of 1 to 4 carbon atoms, or

(ii) $COR_1$ wherein $R_1$ is selected from the group consisting of H, $(CH_2)_nCH_3$, where n=0-6, $(CH_2)_nCOOR_2$ where n=1-4 and $R_2$ is previously defined, $(CH_2)_nN^+(R_3)_4$ wherein n=1-4, and $(CH_2)_nSO_3^-$ where n=1-4,

and pharmaceutically acceptable salts thereof.

One aspect of the method involves administration of a hypoestoxide (herein designated JO-4A) having the formula:

II

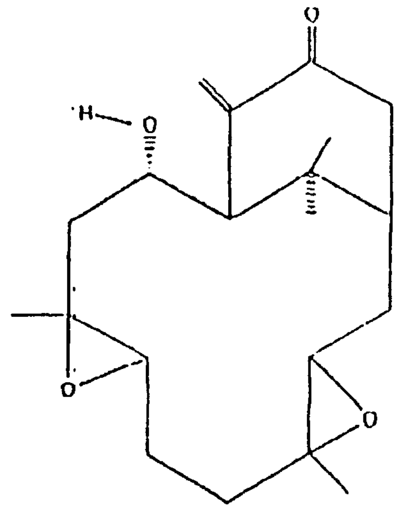

Antiviral Agents. Applicants' invention rests on their finding that a select group of hypoestoxide analogs possess unexpected effectiveness as growth inhibiting agents against lentiviruses and against viruses of the family *Herpetoviridae*, including, respectively, HIV-1 and HSV-1 and HSV-2. In particular, the present invention comprises a method for inhibiting the growth of these viruses in subjects. The method comprises administering to a subject in need of antiviral therapy a pharmaceutical composition comprising a therapeutically effective amount of a compound of the formula

IV

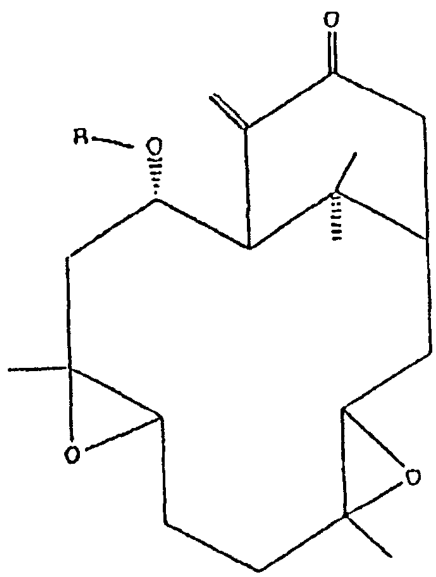

where:

R is

(i) H, $PO_3^=$, alkyl of 1 to 12 carbon atoms substituted or unsubstituted, straight chain or branched, 0 to 6 double bonds, $(CH_2)_n$morpholine where n=1-4, morpholinomethylphenyl, orthoaminophenyl, orthohydroxyphenyl, $(CH_2)_nCOOR_2$ where n =1-4

where $R_2$ is H, an alkalai metal salt, an alkaline earth metal salt, $NH_4^+$, $N^+(R_3)_4$ where $R_3$ is independently selected from the group consisting of H and alkyl of 1 to 4 carbon atoms, or

(ii) $COR_1$ wherein $R_1$ is selected from the group consisting of H, $(CH_2)_nCH_3$, where n=0-6, $(CH_2)_nCOOR_2$

where n=1-4 and $R_2$ is previously defined, $(CH_2)_nN^+(R_3)_4$ wherein n=1-4, and $(CH_2)_nSO_3^-$ where n=1-4,

and pharmaceutically acceptable salts thereof.

In another aspect, the invention provides a method of treating a subject to alleviate pathological effects of the growth of viruses of the lentivirus family (e.g. HIV-1) and *Herpetoviridae family* (e.g. HSV-1 and HSV-2) in the subject. The method comprises administering to the subject at least one hypoestoxide having formula IV. The hypoestoxide is administered to the subject in an amount sufficient to inhibit the growth of these viruses in said subject to thereby inhibit said effects.

The above discussed features and variables of the invention and attendant advantages of the present invention will become better understood by reference to the following detailed description of the invention when taken in conjunction with the accompanying drawings.

## BRIEF DESCRIPTION OF THE DRAWINGS

Figure 1 shows the inhibitory effects of JO-4 on a human two-way mixed lymphocyte reaction. PBMCs from two individuals were co-cultured in the presence of varying concentrations of JO-4 for 5 days. Proliferation was measured by $^3$H-thymidine incorporation and expressed as CPM and % inhibition of controls wells without JO-4.

Figure 2 shows the inhibitory effect of JO-4 on one-way MLR-generated human T-lymphocyte-mediated cytolysis. Cytotoxic T lymphocytes (CTLs) were generated from one-way MLR in the absence or presence of JO-4 for 5-7 days. At the end of culture, CTLs were harvested and tested in a four hour $^{51}$Cr-release assay against activated lymphocytes which were used as stimulators in the one-way MLR. Results are expressed in lytic units (LU) and as % inhibition.

Figure 3 shows the inhibitory effect of JO-4 on pro-inflammatory cytokine synthesis. Human PBMCs were cultured with 5 μg/ml LPS in the absence or presence of 1.0 μg/ml JO-4 in 24-well microtiter plates for 24 hours. At the end of culture, supernatants were harvested and tested by EIA for the presence of IL-1β, TNF-α, and IL-6. The results are expressed as % inhibition of control wells without drug and in pg/ml of cytokine present in the supernatant.

Figure 4 shows the inhibitory effect of JO-4A on human two-way MLR. PBMCs from two individuals were co-cultured in the absence or presence of varying concentrations of JO-4A for 5 days. Proliferation was measured by $^3$H-thymidine incorporation and expressed as mean CPM.

Figure 5 shows the inhibitory effect of JO-4 on LPS-induced murine lymphocyte proliferation *in vitro.* Mouse spleen cells were cultured either in the absence or presence of varying concentrations of JO-4 for 48 hours with LPS (5μg/ml). Proliferation was measured by $^3$H-thymidine incorporation and expressed as ACPM.

Figure 6 shows the inhibitory effect of JO-4 on PHA-induced proliferation of human PBMCs. Human PBMCs were cultured with PHA (15μg/ml) either in the absence or presence of varying concentrations of JO-4 for 4 days. Proliferation was measured by $^3$H-thymidine incorporation and expressed as ACPM and % inhibition of control wells without drug.

Figure 7 shows the toxicity of JO-4 on human PBMCs. Human PBMCs were cultured either in the absence or presence of varying concentrations of JO-4. At different intervals of time, the viability was determined by trypan blue dye exclusion text and expressed as % viability of total number of cells counted.

Figure 8 shows the effects of JO-4A on NK activity *in vivo.* Mice were treated with varying concentrations of JO-4A either by intravenous (A) or oral (B) administration. Spleen cells were tested for NK function using YAC-1 targets in a 4-hour $^{51}$Cr-release assay.

Figure 9 shows the dose-dependent cytotoxic effects of JO-4A on various human cancer cells. Human cervical epitheloid carcinoma, renal carcinoma and malignant melanoma cell lines were cultured for 72 hours either in the presence or absence of various concentrations of JO-4A. Cytotoxicity was determined by colorimetric (MTT) assay.

Figure 10 shows the dose-dependent cytotoxic effects of JO-4A on normal human cells of various tissue origin.

Normal human cervical ectoepithelial cells, peripheral blood mononuclear cells, mammary epithelial cells and umbilical vein endothelial cells were cultured either in the presence or absence of various concentrations of JO-4A for 72 hours. Cytotoxicity was determined by colorimetric (MTT) assay.

Figure 11 shows the dose-dependent cytotoxic effect of JO-4A on mouse malignant melanoma (B16-F1) cell line. B16-F1 cells were cultured either in the presence or absence of various concentrations of JO-4A for 72 hours. Cytotoxicity was determined by colorimetric (MTT) assay.

Figure 12A shows the effect of oral administration of JO-4A on mouse malignant melanoma (B16-F1) volume in C57BL/6 (B6) mice. B6 mice received 50,000 B16-F1 viable cells subcutaneously (s.c.). Mice in the experimental groups received different concentrations of JO-4A orally everyday starting from day 4 after tumor implantation. Tumor volume was measured on day 12 using a microcaliper.

Figure 12B shows the effect of oral administration of JO-4A on the survival of malignant melanoma (B16-F1) bearing B6 mice. B6 mice received 50,000 B16-F1 viable cells subcutaneously (s.c.). Mice in the experimental groups received different concentrations of JO-4A orally everyday from day 4 post tumor implantation to day 20. Percent survival of mice in the experimental groups was calculated when all the mice in the non-treated control group were dead (day 28).

Figure 13 shows the effect of oral administration of JO-4A on the survival of B16-F1 melanoma bearing B6 mice. B6 mice received 50,000 B16-F1 viable cells subcutaneously (s.c.). Mice in the experimental group received JO-4A (2 mg/Kg) orally everyday from day 4 to day 20.

Figure 14 shows the effect of JO-4A in combination with IFN-alpha on the growth of human malignant melanoma *in vitro*.

Figure 15A shows the inhibitory effect of JO-4A on HIV-1 replication in cultured human peripheral blood mononuclear cells (PBMC). Human PBMCs were activated with PHA and infected with HIV-1 isolate from a patient. Infected PBMCs were cultured for 7 days either in the absence or presence of varying concentrations of JO-4A. (0.0001 μM - 0.5 μM). At the end of culture, supernatants were collected and measured by ELISA for the presence of p24 antigen.

Figure 15B shows the toxicity testing of JO-4A on cultured normal human PBMCs, which have been activated with PHA for 3 days. Activated PBMCs were cultured with 3% IL-2 for 7 days either in the absence or presence of varying concentrations of JO-4A. (0.0001 μM - 0.5 μM). At the end of culture, the viability of the PBMCs were determined by trypan blue dye-exclusion test.

Figure 16A shows the antiviral effect of JO-4 on *Herpes simplex-I* (HSV-1) replication. Antiviral effects of JO-4 on HSV-1 were determined using Hybriwix Probe Systems: HSV antiviral susceptibility test kit from Diagnostic Hybrids, Inc. (Athens, OH).

Figure 16B shows the antiviral effect of JO-4 on HSV-2 replication. Antiviral effects of JO-4 on HSV-2 were determined as described in Figure 16A.

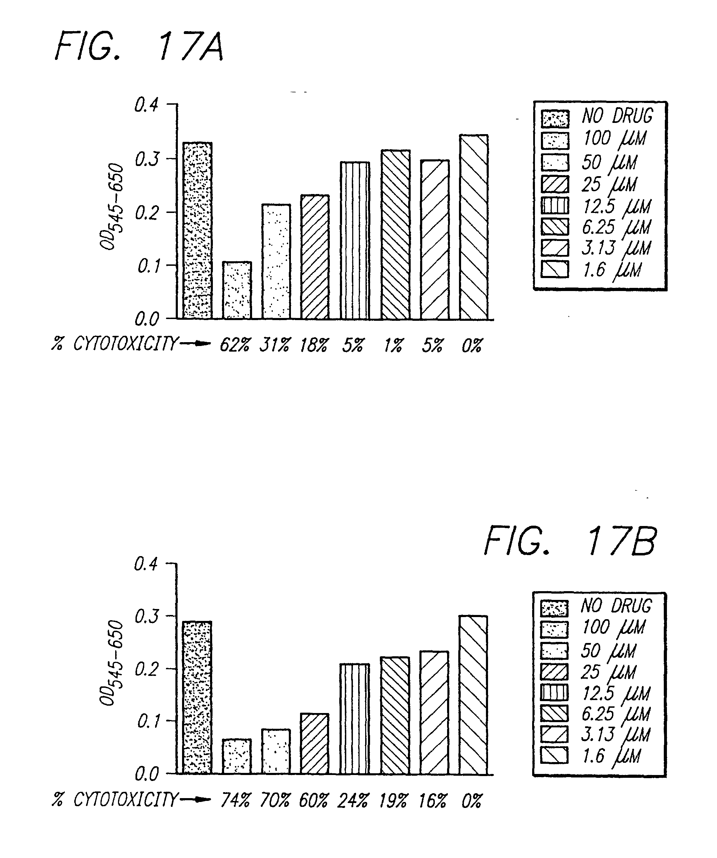

Figure 17A and 17B show the results of toxicity testing of JO-4 and JO-4A on normal, uninfected African green monkey kidney cells (CV-1), respectively. CV-1 cells were cultured either in the presence or absence of various concentrations of drugs for 72 hours. Cytotoxicity was determined by colorimetric (MTT) assay.

## MODES OF CARRYING OUT THE INVENTION

### General Description and Definitions

The practice of the present invention will employ, unless otherwise indicated, conventional molecular and cellular immunology, cell biology, biochemistry, and organic and medicinal chemical synthesis within the skill of the art. Such techniques are explained fully in the literature. See, e.g. Abbas, A.K., et al., eds, 1994, 2nd ed. Cellular and Molecular Immunology, W.B. Saunders Co., USA; Harlow, E. and D. Lane, Antibodies, A Laboratory Manual, Cold Spring Harbor Laboratory, 1988; Auchinclos, Jr., H. and Sachs, D.H. Transplantation and Graft Rejection in: Fundamental Immunology, Paul, W.E. ed., 1993, Raven Press; Silverman, Richard B., The Organic Chemistry of Drug Design

and Drug Action, Academic Press, Inc. NY (1992); Smith, Michael B., Organic Synthesis, McGraw Hill, Inc., NY, (1994)). Also see Cancer Chemotherapy: Principles and Practice, ed. B.A. Chabner, J.M. Collins, Phil., Lippincott Publ., 1990; Mechanisms of Interferon Actions, Vol. II, ed. L.M. Pfeffer, CRC Press, Boca Raton, FL 1987; Interferons: Principles and Medical Applications, ed. S. Baron, D. Coppenhauer, F. Dianzani, et al., The University of Texas Medical Branch, Galveston, 1992; Robins, R.K., Ojo-Amaize, E.A., Cottam, H.B., et al., Nucleoside and nucleotide modulation of genetic expression: A new approach to chemotherapy. In: Advances in Enzyme Regulation, 1989, Vol. 29; Cancer: Principles and Practice of Oncology; ed. de Vita, Jr., V.T., Hellman, S., and Rosenberg, S.A., Lippincott Co., Philadelphia, 1993; The Chemotherapy Source Book, ed. Perry, M.C., Williams and Wilkins Publ., Baltimore, 1991). Silverman, Richard B., The Organic Chemistry of Drug Design and Drug Action, Academic Press, Inc. NY (1992); Smith, Michael B., Organic Synthesis, McGraw Hill, Inc., NY, (1994)). Further see Bauer, D.J., The Specific Treatment of Virus Diseases, University Park Press, Baltimore, MD, 1977; Gawler, H., Nucleic Acid Hybridization for Measurement of Antiviral Compounds on Human Cytomegalvirus DNA Replication, Antimicrobial Agents Chemotherap, 1983, 24:370-374; Collier, L.H. and Oxford, J., eds., Developments in Antiviral Therapy, Academic Press, London, 1980; Coligan, J.E. et al., eds., Current Protocols in Immunology, 1993; Robert B. Belshe, ed., Textbook of Human Virology, 2nd. ed., 1991; Silverman, Richard B., The Organic Chemistry of Drug Design and Drug Action, Academic Press, Inc. NY (1992); Smith, Michael B., Organic Synthesis, McGraw Hill, Inc., NY, (1994)).

Immunosuppression. The following terminology will be used in accordance with the definitions set out below in describing the present invention.

For purposes of the invention, the term "immunosuppressive treatment" refers to an approach to the prevention and management of diseases and syndromes, which require for therapy the suppression of lymphocytes and immunocytes. Such diseases and syndromes include, but are not limited to, graft vs. host disease (GVHD), autoimmune diseases such as diabetes, rheumatoid arthritis, systemic lupus erythematosus, thyroiditis, multiple sclerosis, glomerulonephritis, and allergy. It is understood that a commonly used method of immunosuppression involves administering to an animal in need of such treatment a therapeutically effective amount of an immunosuppressive agent (e.g. cyclosporin A, FK506, or the compositions and methods exemplified and claimed herein) in order to inhibit T-cell activity or T-cell response.

The terms "lymphocytes and immunocytes" refer to cells that mediate the specificity of immune responses. As used herein, the terms refer to T lymphocytes, which are described in detail in Abbas et al.

"Natural killer cells" or "NK cells" are non-T, non-B lymphocytes usually having granular morphology. NK cells are important in innate immunity to viruses and other intracellular pathogens as well as in antibody-dependent cell-mediated cytotoxicity (ADCC). NK cells are responsible in large degree for graft versus host disease (GVHD) (Ferrara, J.L.M., et al. (1989) Transplantation 47:50-54; Ghayur, T. et al. (1987) Transplantation 44:261-267; Ghayur, T., et al. (1988) Transplantation 45:586-590. As demonstrated in the Examples below, it was found that the compounds and methods of the invention suppressed NK cell activity *in vivo* and found use in preventing or overcoming GVHD in animals in need of such treatment.

The term "transplantation" refers to the process of taking cells, tissues, or organs, called a graft, from one individual and, usually, placing them into a different, genetically non-identical recipient. The individual who provides the graft is referred to as the donor, and the individual who receives the graft is referred to as either the recipient or host. Transplantation leads to a specific immune response, called rejection, that can destroy the graft. Transplant tissues include solid organs, such as liver, heart, and kidneys; and other tissues such as skin and bone marrow. *In vivo* rejection is mediated by T cells. Rejection may be prevented or treated by immunosuppressing the recipient (host) by administering to the host therapeutically effective amounts of compounds such as cyclosporin A or FK506, as well as administering the compositions exemplified herein. Most immunosuppression is directed at T-cell responses using specific immunosuppressive agents, not unlike the compositions of the present invention. In addition, immunosuppression of NK cell function in donors plays an important therapeutic role in treating conditions such as graft versus host disease (GVHD). Accordingly, transplant donors require preparatory immunosuppression by administration of a therapeutically effective amount of an immunosuppressive agent prior to donating a transplant for prevention of graft versus host disease in a recipient.

The term "animals" is taken to mean humans as well as other animals.

As used herein, the term "JO-4" means a compound which is a bicycle [9,3,1] pentadecane diterpene compound, as described in Z. Naturforsc 37 c: 558-561 (1982) and in Heterocycles 20:2125-2128 (1983), in which reference this compound is named "hypoestoxide." The chemical structure of JO-4 is illustrated in formula I.

It is understood that the compounds illustrated in formula IV include prodrugs of JO-4A. In terms of formula IV, JO-4A is derived from JO-4 when R is H. The structure of JO-4A is illustrated in formula II.

II

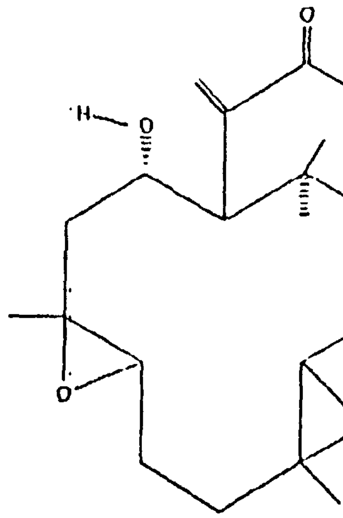

The term "prodrug," as used herein, refers to a pharmacologically inactive compound that is converted to an active drug by a metabolic transformation. (Silverman, Richard B. The Organic Chemistry of Drug Design, Acad. Press, 1992). There are numerous reasons why a prodrug strategy is used in drug design, the most common of which are to overcome problems associated with the compound, such as solubility, absorption and distribution, site specificity, instability, prolonged release, toxicity, poor patient acceptability, and formulation. Literature is available for guidance without undue experimentation for determining how to get compounds in pharmaceutical compositions to a locus to permit them to act, and guidance for how to obtain an immunosuppressive therapeutic effective amount at the locus of action (Harnden, M.R. et al., J. Med. Chem. 32:1738-1743 (1989); Lake-Bakaar, D.M., et al, Antimicrobial Agents and Chemotherapy 33:110-112 (1989); Baker, D.C., et al. J. Medicinal Chemistry 21:1218-1221 (1978); Hussain, M. A. et al., J. Pharmaceutical Sciences, 76:356-358 (1987); Varia, S.A. et al., J. Pharmaceutical Sciences 73:1068-1073 (1984)).

The most common prodrug form for drugs containing alcohol or carboxylic acid functional groups is an ester. Using skills well known in the art, it is possible to alter the structure of the compound to improve its pharmacokinetic properties and, thereby, transform it into a useful drug for therapeutic administration to an animal or human. Accordingly, the rationale for medicinal activity (i.e. inducing immunosuppression) of the claimed prodrugs of JO-4A is that JO-4 itself, the natural product, is a potent immunosuppressive agent. JO-4 is also a prodrug for JO-4A in the presence of serum esterases in the *in vivo* setting, and, in the *in vitro* setting if the culture medium contains added serum (which is most often the case). A preferred embodiment of the immunosuppressant agent is the metabolite JO-4A, which is the free alcohol derivative of JO-4. JO-4 serves as an ester prodrug form for the delivery of JO-4A, which is formed over time after administration of JO-4 to cells or animals. In similar fashion, many other ester prodrugs of JO-4A provide delivery of JO-4A. Such prodrug forms and methods for making them are well known in the art, as cited above. These prodrugs are known to yield the parent drugs of interest upon exposure to esterases commonly found in serum of animals and humans. It is understood that the prodrugs of JO-4A claimed herein yield JO-4A and are as active or more active in terms of inducing immunosuppression than the natural product JO-4.

The term "agonists" as used herein refers to substances that elicit the same response (i.e. inducing immunosuppression in animals in need of such treatment) as the compounds indicated in formula IV. Agonists of the compounds of formula IV include, but are not restricted to the prodrugs of JO-4A, which prodrugs are illustrated in formula IV.

Methods for determining or screening modified forms of the claimed compounds, i.e. prodrugs and/or agonists of the claimed compounds, for their ability to induce immunosuppression in animals, or for their ability to inhibit growth of cancer in animals, or for their ability to inhibit the growth of lentiviruses or *Herpetoviridae* viruses in animals in need of such treatment are well known in the art.

The methods of the present invention are directed to immunosuppressive therapy using the compounds of formula IV, as well as the compounds of formula I (JO-4, i.e. hypoestoxide). In particular, the methods of the present invention involve administering to an animal in need of such treatment a therapeutically effective amount of the compounds of formulas I or IV. An embodiment of the methods involves associating compounds of formulas I or IV with a pharmaceutical carrier or diluent for administration to an animal.

The method of the invention finds use in suppressing the rejection of transplants in animals, as illustrated by the effects of JO-4 or JO-4A on cellular immunity in the one-way and two-way MLR tests set forth below. Thus, the

method of the invention is useful in the suppression of the formation of, or proliferation of immunocytes or lymphocytes, and is therefore useful in the treatment of autoimmune diseases, and in suppressing the rejection of transplants, e.g. organ transplants, such as skin, bone marrow, kidney, liver and heart transplants. The effects of JO-4 or JO-4A were ascertained in the tests described below.

As indicated by Figures 1-8, compounds and the method of the invention find use in treating autoimmune diseases in animals. It is understood that immunosuppressants for T-cells, such as cyclosporin A (Forsell, T et al., J. Urol, 1996, 5:1591-3; Yocum, D.E., et al., Rheum. Dis. Clin. North. Am., 1995, 3: 835-44) or tacrolimus (Ketel, B.L., et al., Transplant. Proc., 1996, 2:899) suppresses immune reactions and result in inhibition or amelioration of autoimmune disorders. T-cells and their products (cytokines) are known to be involved in the generation and production of autoimmune reactions *in vivo.*

For all of the above-mentioned uses, the therapeutic effective amount or dosage will, of course, vary depending on the compound employed, mode of administration and treatment desired. However, in general, satisfactory results would be obtained when administered orally at a daily dosage of from about 1 mg to about 600 mg per kg animal body weight, conveniently given in divided doses 1 to 4 times a day or in sustained release form. If administered by injection, in general, satisfactory results would be obtained when administered at a daily dosage of from about 1 mg to about 200 mg per kg animal body weight, conveniently given in divided doses 1 to 4 times a day or in sustained release form. For the larger mammals, the total daily dosage would be in the range of from about 5 to about 500 mg, and dosage forms suitable for oral administration comprise from about 5 mg to about 500 mg of the compounds admixed or in association with a solid or liquid pharmaceutical carrier or diluent. Methods are well known in the art for determining therapeutically effective amounts of the compounds of the invention. Such methods involve analysis of the pharmaceutical/pharmacokinetic parameters in immunosuppressive therapy for inducing immunosuppression, for suppressing formation of lymphocytes and immunocytes, for treating autoimmune diseases, and for suppressing rejection of transplants in animals or other indications ( Recent Developments in Transplantation Medicine, Vol. 1: New Immunosuppressive Drugs: eds. D. Przepiorka and H. Sollinger, Physicians and Scientists Pub. Co. , Glenview, IL, 1994).

The method of the present invention includes administering a pharmaceutical composition comprising an effective amount of the compounds of the formulas I, II or IV in pure form or as a pharmaceutically acceptable crude concentrate in association with a pharmaceutical carrier or diluent. Such compositions conveniently contain more than 1% by weight of the compounds of formulas I, II or IV and may be prepared by conventional techniques to be in conventional forms, for example, capsules, tablets, suppositories, dispersible powders, syrups, elixirs, suspensions or solutions for enteral or parenteral administration. Suitable pharmaceutical diluents or carriers include, for example, water, alcohols, natural or hardened oils and waxes, calcium and sodium carbonates, calcium phosphate, kaolin, talc and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan mono-oleate, granulating and disintegrating agents such as starch and alginic acid, binding agents such as starch, gelatin and acacia and lubricating agents such as magnesium stearate, stearic acid and talc, in order to provide an elegant and palatable pharmaceutical preparation. Compositions in tablet form may be coated by conventional techniques to delay disintegration of the tablet and absorption of the active ingredient in the gastrointestinal tract and thereby provide sustained action over a long period. Other compounds and methods known in the art for delaying disintegration or for timed-delayed or time-measured delivery of the active ingredients also find use in formulating the active ingredients for use in the methods of the invention. For example, the compounds of formulas I, II or IV may also be combined with liposomes or other delayed-release carrier means to protect the compounds from degradation until they reach their targets and/or facilitate movement of the compounds across tissue barriers.

The preferred compositions from the standpoint of ease of administration are solid compositions, particularly solid-filled gelatin capsules or tablets.

It is also to be understood that a further embodiment of the method of the invention involves combining one or more agents in a variety of protocols, including prophylaxis, with the method of the invention for administering to animals in need of immunosuppressive treatment pharmaceutical compositions comprising compounds of formulas I, II or IV. Combination protocols and methods for determining their efficacy, including therapeutic drug monitoring, are well known in the art (Lazarus, H.M. et al. Blood Reviews, 1995, 9:117-133; Aggarwal, B.B., et al., eds., 1995, Human Cytokines: Their role in Disease and Therapy, Blackwell Science, Inc., Cambridge, MA; Ambrus, J.L. et al. , 1993, Surgery, Gynecology & Obstetrics, 176:588). Examples of immunosuppressive agents or agents useful in immunosuppressive therapy which may be combined with administering the compounds of formulas I, II or IV in the method of the invention include, but are not limited to corticosteroids, methotrexate, cyclosporin, rapamycin, FK506 (Prograf$^{tm}$), antithymocyte globulin, monoclonal antibody preparations, polyclonal antibody preparations, interleukin 1 antagonists, interleukin 2 antagonists, TNF antagonists, immunotoxins, thalidomide, interferon, nitric oxide, mizoribine, deoxyspergualin, leflunomide, and anti-adhesion molecules.

It will be further understood that the present invention includes a pharmaceutical composition comprising a therapeutically effective amount of one or more compounds of formulas I, II or IV in association with one or more agents

selected from the group of immunosuppressive agents or agents useful in immunosuppressive therapy consisting of corticosteroids, methotrexate, cyclosporin, rapamycin, FK506 (Prograftm), antithymocyte globulin, monoclonal antibody preparations, polyclonal antibody preparations, interleukin 1 antagonists, interleukin 2 antagonists, TNF antagonists, immunotoxins, thalidomide, interferon, nitric oxide, mizoribine, deoxyspergualin, leflunomide, and anti-adhesion molecules. Methods are well known in the art for determining therapeutically effective amounts of the compounds of formulas I, II or IV, and agents selected from the group of immunosuppressive agents or agents useful in immunosuppressive therapy in association with the compounds of formulas I, II or IV in pharmaceutical compositions of the invention ((Lazarus, H.M. et al. Blood Reviews, 1995, 9:117-133; Aggarwal, B.B., et al., eds., 1995, Human Cytokines: Their role in Disease and Therapy, Blackwell Science, Inc., Cambridge, MA; Ambrus, J.L. et al., 1993, Surgery, Gynecology & Obstetrics, 176:588).

Anticancer. The following terminology will be used in accordance with the definitions set out below in describing the present invention.

Although "tumor" simply means "swelling", the term is usually equated with "neoplasm", which literally means "new growth". A neoplasm is an abnormal mass of tissue that persists and proliferates after withdrawal of the carcinogen that initiated its appearance. There are two types of neoplasms or tumors, benign and malignant. The common term for all malignant tumors is "cancer". Nearly all benign tumors are encapsulated. In contrast, cancerous tumors are almost never encapsulated but invade adjacent tissue by infiltrative destructive growth. Invasive growth may be followed by tumor cells implanting at sites discontinuous with the original tumor usually through lymphatic and/or hematogenous spread of the cancer cells. This process, called "metastasis," unequivocally marks a tumor as malignant because a benign tumor never metastasizes whereas most cancers can metastasize (In: Pathologic basis of disease, 4th ed. Philadelphia: WB Saunders, 1989;239-305). It will be understood that the present method for inhibiting the growth of cancer cells or cancerous tumors includes inhibiting invasive growth of cancer cells as well as inhibiting metastatic growth of tumors from metastisized cancer cells.

The term "subject" is taken to mean humans as well as other animals.

As used herein, the term "JO-4" means a compound which is a bicycle [9,3,1] pentadecane diterpene compound, as described in Z. Naturforsc 37 c: 558-561 (1982) and in Heterocycles 20:2125-2128 (1983), in which reference this compound is named "hypoestoxide." The chemical structure of JO-4 is illustrated in formula 1.

I

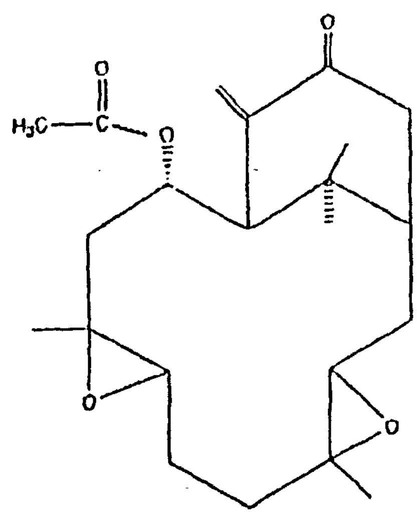

It is understood that the compounds illustrated in formula IV include prodrugs of JO-4A. In terms of formula IV, JO-4A is derived from JO-4 when R is H. The structure of JO-4A is illustrated in formula II.

II

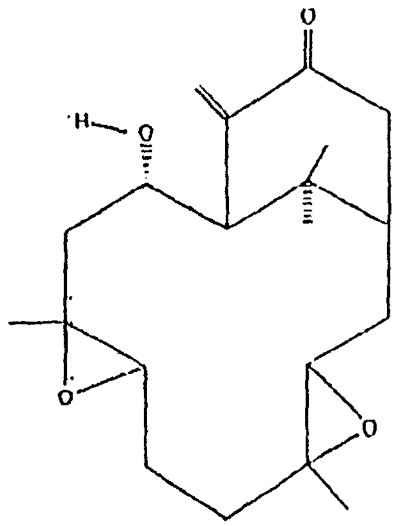

The term "prodrug," as used herein, refers to a pharmacologically inactive compound that is converted to an active drug by a metabolic transformation. (Silverman, Richard B. The Organic Chemistry of Drug Design, Acad. Press, 1992). There are numerous reasons why a prodrug strategy is used in drug design, the most common of which are to overcome problems associated with the compound, such as solubility, absorption and distribution, site specificity, instability, prolonged release, toxicity, poor patient acceptability, and formulation. Literature is available for guidance without undue experimentation for determining how to get compounds in pharmaceutical compositions to a locus to permit them to act, and guidance for how to obtain a therapeutically effective amount for inhibiting the growth of cancerous tumors at the locus of action (Brunda, M.J., Wright, R.B., Luistro, L. et al., Enhanced Antitumor Efficacy in Mice by Combination Treatment with Interleukin-1α and Interferon-α, J. Immunother., (1994) 15:233-241; Bezwoda, W.R., The Treatment of Disseminated Melanoma with Special Reference to the Role of Interferons, Vinca Alkaloids, and Tamoxifen, Cancer Treatment Review, (1997) 23:17-34; Wedge, S.R., Porteous, J.K., Newlands, E.S., Effect of Single and Multiple Administration of an $O^6$-benzylguanine/temozolomide Combination: An evaluation in a Human Melanoma Xenograft Model, Cancer Chemother. Pharmaocol. (1997) 40: 266-272).

The most common prodrug form for drugs containing alcohol or carboxylic acid functional groups is an ester. Using skills well known in the art, it is possible to alter the structure of the compound to improve its pharmacokinetic properties and, thereby, transform it into a useful drug for therapeutic administration to an animal or human. JO-4 is a prodrug for JO-4A in the presence of serum esterases in the in vivo setting, and, in the *in vitro* setting if the culture medium contains added serum (which is most often the case). A preferred embodiment of the hypoestoxide compound for use in the method for inhibiting the growth of cancerous tumors is the metabolite JO-4A, which is the free alcohol derivative of JO-4. JO-4 serves as an ester prodrug form for the delivery of JO-4A, which is formed over time after administration of JO-4 to cells or animals. In similar fashion, many other ester prodrugs of JO-4A provide delivery of JO-4A. Such prodrug forms and methods for making them are well known in the art, as cited above. These prodrugs are known to yield the parent drugs of interest upon exposure to esterases commonly found in serum of animals and humans. It is understood that the prodrugs of JO-4A useful in the claimed method yield JO-4A and are active in terms of inhibiting the growth of cancerous tumors.

The term "agonists" as used herein refers to substances that elicit the same response (i.e. inhibiting the growth of cancerous tumors in subjects in need of such treatment) as the compounds indicated in formula IV. Agonists of the compounds of formula IV include, but are not restricted to the prodrugs of JO-4A, which prodrugs are illustrated in formula IV.

Methods for determining or screening modified forms of the hypoestoxide compounds i.e. prodrugs and/or agonists of the claimed compounds, for their ability to inhibit the growth of cancerous tumors in subjects in need of such treatment are well known in the art (Stinson, S., Alley, M.C., Kopp, W.C. et al. , Morphological and Immunocytochemical Characteristics of Human Tumor Cell Lines for Use in a Disease-Oriented Anticancer Drug Screen, Anticancer Research (1992) 12:1035-1054).

The method of the present invention is directed to anticancer or antitumor therapy, i.e. inhibiting the growth

of cancer cells, and thereby, when the growth of cancer cells results in the development of one or more tumors, inhibiting the growth of cancerous tumors using the compounds of formula IV, and in particular, the compounds of formula II (JO-4A, i.e. hypoestoxide). In particular, the method of the present invention involve administering to a subject in need of such treatment a therapeutically effective amount of at least one hypoestoxide compound of formulas IV, and in particular the compound of formula II (JO-4A). An embodiment of the method involves associating compounds of formulas IV or, in particular, II with a pharmaceutical carrier or diluent for administration to an subject.

The method of the invention finds use in inhibiting the growth of cancerous cells *in vitro*, as illustrated in Figure 9, and in inhibiting the growth of cancer cells or cancerous tumors in mice, as shown in Figures 12 and 13. Thus, the method of the invention finds use in having a cytotoxic , growth-inhibiting effect on cancerous cells or cancerous tumors, and, at the same dosage level, having a relatively non-toxic effect on normal cells (Figure 10), which differential toxic effect appears to inhibit the growth of cancerous tumors, and is therefore useful in anti-tumor treatment at least for the variety of cancer types indicated in Figure 9. The effects of JO-4A were ascertained in the tests described below, as illustrated in Figures 9-13, showing that the method of the invention is effective for treating subjects in need of anticancer therapy.

For the above-mentioned use, the therapeutic effective amount or dosage will, of course, vary depending on the compound employed, mode of administration and treatment desired. However, in general, satisfactory results would be obtained when administered orally at a daily dosage of from about 0.01 mg to about 1000 mg per kg animal body weight, conveniently given in divided doses 1 to 4 times a day or in sustained release form. If administered by intravenous injection, in general, satisfactory results would be obtained when administered at a daily dosage of from about 0.01 mg to about 200 mg per kg animal body weight, conveniently given in divided doses 1 to 4 times a day or in sustained release form. For the larger mammals, the total daily dosage would be in the range of from about 1 to about 1000 mg, and dosage forms suitable for oral administration comprise from about 1 mg to about 1000 mg of the compound admixed or in association with a solid or liquid pharmaceutical carrier or diluent. Methods are well known in the art for determining therapeutically effective amounts of the compounds used in the method of the invention. Such methods involve analysis of the pharmaceutical/pharmacokinetic parameters in anti-cancer or antitumor therapy, i.e for inhibiting the growth of cancerous tumors (Wedge. S.R., Porteus, J.K., Newlands, E.S., Cancer Chemother. Pharmacol. (1997) 40:266-272; Legha, S.S., Seminar in Oncology, (1997) 24:S4-24-31; Motzer, R.J., Vogelzang, N.J., Chemotherapy for Renal Cell Carcinoma. In: Raghaven, D., Scher, H.I., Leibel, S.A., et al: eds. Principles and Practice of Genitourinary Oncology, Lippincott-Raven Publ., Philadelphia, pp. 885-96, 1997; Bloom, H.J., Medroxyprogesterone acetate (Provera) in the treatment of metastatic renal cancer, Br. J. Cancer (1971) 25:250-65)

The method of the present invention includes administering a pharmaceutical composition comprising an effective amount of one or more of the compounds of formula IV, and in particular the compound of formula II (JO-4A) in pure form or as a pharmaceutically acceptable crude concentrate in association with a pharmaceutical carrier or diluent. Such compositions conveniently contain less than 1% by weight, and preferably about 0.2% by weight, of the compounds of formulas IV or, in particular, II, and may be prepared by conventional techniques to be in conventional forms, for example, capsules, tablets, suppositories, dispersible powders, syrups, elixirs, suspensions or solutions for enteral or parenteral administration. Suitable pharmaceutical diluents or carriers include, for example, water, alcohols, natural or hardened oils and waxes, calcium and sodium carbonates, calcium phosphate, kaolin, talc and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan mono-oleate, granulating and disintegrating agents such as starch and alginic acid, binding agents such as starch, gelatin and acacia and lubricating agents such as magnesium stearate, stearic acid and talc, in order to provide an elegant and palatable pharmaceutical preparation. Compositions in tablet form may be coated by conventional techniques to delay disintegration of the tablet and absorption of the active ingredient in the gastrointestinal tract and thereby provide sustained action over a long period. Other compounds and methods known in the art for delaying disintegration or for timed-delayed or time-measured delivery of the active ingredients also find use in formulating the active ingredients for use in the methods of the invention. For example, the compounds of formulas IV or in particular II may also be combined with liposomes or other delayed-release carrier means to protect the compounds from degradation until they reach their targets and/or facilitate movement of the compounds across tissue barriers.

The preferred compositions from the standpoint of ease of administration arc solid compositions, particularly solid-filled gelatin capsules or tablets.

It is also to be understood that a further embodiment of the method of the invention involves combining one or more agents in a variety of protocols, including prophylaxis, with the method of the invention for administering to subjects in need of treatment for inhibiting the growth of cancerous tumors pharmaceutical compositions comprising compounds of formulas IV or, in particular, II. Combination protocols and methods for determining their efficacy, including therapeutic drug monitoring, are well known in the art. Examples of anti-cancer agents and other agents useful in therapy for inhibiting the growth of cancerous tumors which may be combined with administering the compounds of formulas IV or in particular II in the method of the invention include, but are not limited to radiation, interferon-a, interleukin-1, -2, vinca alkaloids, tamoxifen, taxol, decarbazine, biological response modifiers, platinum compounds, bleomycin, dipyridamole, carmustine, cisplatin.

It will be further understood that the present invention includes a method for inhibiting the growth of cancerous tumors which comprises administering to a subject in need of anticancer treatment pharmaceutical composition comprising a therapeutically effective amount of one or more compounds of formulas IV and/or II (JO-4A) in association with one or more agents selected from the group of anti-cancer agents or agents useful in inhibiting or preventing growth of cancerous tumors consisting of radiation, interferon-$\alpha$, interleukin-1, -2, vinca alkaloids, tamoxifen, taxol, decarbazine, biological response modifiers, platinum compounds, bleomycin, dipyridamole, carmustine, cisplatin. Methods are well known in the art for determining therapeutically effective amounts of the compounds of formulas IV or II and agents selected from the group of anti-cancer agents or agents useful in anti-cancer therapy in association with the compounds of formulas IV or II in pharmaceutical compositions in the method of the invention (William, S.D., ed., Current Problems in Cancer, Vol. 21, No. 4,1997; pp. 186-221; Bezwoda, W.R., Cancer Treatment Reviews, 1997, Vol. 23: 17-34)

Antiviral. The following terminology will be used in accordance with the definitions set out below in describing the present invention.

The term "inhibiting the growth of" is used with respect to lentiviruses and *Herpetoviridae* viruses that are pathological to human or other mammals. For example, with respect to the lentivirus HIV, "inhibiting the growth of" means inhibition of virus production as determined by decreased HIV $p^{24}$ levels in virus culture. Inhibiting the growth of HIV results on reduction of virus load, which, in turn, alleviates the pathological effects of HIV infection. It will be understood that as used herein, "inciting the growth of" effectively reduces viral load for a DNA or RNA virus of interest, which in turn, alleviates the pathological effects of infection by that DNA or RNA virus.

The term "pathological effects" as used herein is illustrated by an understanding of the life cycle of the lentivirus HIV and its effects in a host. It will be understood that lentiviruses include HIV-1, HIV-2, and HTLV, and that the method of the invention is directed for inciting the growth of HIV-1, HIV-2 or HTLV in subjects. The human immunodeficiency virus type 1 (HIV-1), a retrovirus, is a single-stranded RNA genome is packaged inside a protein core particle and surrounded by a lipid envelope in which is embedded the outer coat (envelope) protein, $gp^{120}$. Infection of T-helper ($CD4^+$) lymphocytes and monocytes begins with adsorption of virions to the cell surface mediated by the specific interaction of the virus envelope protein with CD4 molecules on the cell surface. After viral entry, the virus uncoats and the duplicate, single-stranded RNA genome is reverse-transcribed into a double-stranded DNA genome by the viral enzyme reverse transcriptase (RT). Integration into the host DNA is followed by transcription and translation of HIV-1 genes (Arch. Intern. Med., 1997, 157:951-959).

Pathological/cytopathic effects of HIV consist, in part, of cell fusion with formation of syncitia and subsequent cell death (Belshe, R.B., ed., Textbook of Human Virology, 2nd ed., 1991), resulting in development of immunodeficiency. AIDS (acquired immunodeficiency syndrome) prestages include lymphadenopathy syndrome and the appearance of constitutional symptoms (AIDS-related complex or ARC). Lymphadenopathy syndrome is defined as enlargement of lymph nodes with any other recognizable cause other than HIV infection. Opportunistic infections comprise diseases with mostly ubiquitous parasites which are harmless in immunocompetent persons and acquire pathogenicity only in the presence of immunodeficiency. A high percentage of HIV-infected patients shows neurologic changes that are not explained by opportunistic infections or tumors. Dermatologic manifestations are frequently observed. Herpes zoster is an early clinical sign.

The family *Herpetoviridae* has several members which are widely disseminated human pathogens. These include herpes simplex virus (HSV), Epstein-Barr virus (EBV), cytomegalovirus (CMV), and varicella-zoster virus. It will be understood that the method of the invention is directed to inhibiting the growth *herpetoviridae* viruses including HSV-1 or -2, EBV, CMV and varicella-zoster. HSV, types 1 and 2, have been investigated in great detail at the molecular level because of their rapid replication cycle and high yields in tissue culture (Belshe, R.B., ed., Textbook of Human Virology, 2nd ed., 1991). In relation to HSV, the term "inciting the growth of" means inhibition of replication cycle, diminishing high yield or production of virus. Replication cycle involves: attachment, penetration, uncoating, early transcription, DNA replication, late transcription, and virus assembly. Pathological effects result from replication when host cells are infected. HSV infection alters cell organization at two levels, namely, changes in intracellular structures as well as in cell interactions. Cell protein and DNA synthesis are inhibited by viral protein(s). Chromosomes fragment during infection and remnants relocate to the inner surface of the nuclear membrane, thereby clearing the center of the nucleus for viral replication. At a different level, interactions between cells are altered as a result of HSV infections in a manner influenced by viral strain or cell type or both. This is shown by varying degrees of aggregation of rounded cells and cell fusions. Cultured cells infected with variants of HSV often exhibit altered cell-cell associations, with increased clumping of syncytia formation.

Pathological effects of HSV type 1 result in Herpes simplex encephalitis (HSE), which is a life-threatening condition with high mortality and significant morbidity in survivors. Acute focal, necrotizing encephalitis including inflammation and swelling of the brain tissue with petechiae leading to larger hemorrhages are consistent features of

the pathology of HSE (Scand. J. Infect. Dis Suppl. 100:8-13, 1996). Pathological effects of HSV 2 result in genital ulceration in developed countries. Herpes viruses, which are endemic in all human populations, include herpes simplex virus, varicella-zoster virus, EBV, CMV, and human Herpes viruses-6, 7, and 8.

As used herein, the term "alleviate" means to lessen or reduce or make more bearable.

The term "subject" is taken to mean humans as well as other animals.

The term "prodrug," as used herein, refers to a pharmacologically inactive compound that is converted to an active drug by a metabolic transformation. (Silverman, Richard B. The Organic Chemistry of Drug Design, Acad. Press, 1992). There are numerous reasons why a prodrug strategy is used in drug design, the most common of which are to overcome problems associated with the compound, such as solubility, absorption and distribution, site specificity, instability, prolonged release, toxicity, poor patient acceptability, and formulation. Literature is available for guidance without undue experimentation for determining how to get compounds in pharmaceutical compositions to a locus to permit them to act, and guidance for how to obtain a therapeutically effective amount for inhibiting the growth of pathological RNA or DNA viruses, including but not limited to HIV or HSV, respectively, at the locus of action (McDonald, C.K., Kuritzkes, D.R., Human Immunodeficiency Virus Type 1 Protease Inhibitors, Arch. Intern. Med., 1997, 157: 951-959); Klagstaff, A.J., Faulds, D., Goa, K.L., Aciclovir: A Reappraisal of its Antiviral Activity, Pharmacokinetic Properties, and Therapeutic Efficacy, Drugs, 1994, 47(1):153-205; Hayashi, K., et. al., Characterization of Antiviral Activity of Sesquiterpene, triptofordin, J. Antimicrob. Chemother., 1996, 37:759-768).

The most common prodrug form for drugs containing alcohol or carboxylic acid functional groups is an ester. Using skills well known in the art, it is possible to alter the structure of the compound to improve its pharmacokinetic properties and, thereby, transform it into a useful drug for therapeutic administration to an animal or human. JO-4 is a prodrug for JO-4A in the presence of serum esterases in the *in vivo* setting, and, in the *in vitro* setting if the culture medium contains added serum (which is most often the case). A preferred embodiment of the hypoestoxide compound for use in the method for inhibiting the growth of HIV or HSV in subjects to alleviate the pathological effects of the growth of HIV or HSV in subjects is the metabolite JO-4A, which is the free alcohol derivative of JO-4. JO-4 serves as an ester prodrug form for the delivery of JO-4A, which is formed over time after administration of JO-4 to cells or animals. In similar fashion, many other ester prodrugs of JO-4A provide delivery of JO-4A. Such prodrug forms and methods for making them are well known in the art, as cited above. These prodrugs are known to yield the parent drugs of interest upon exposure to esterases commonly found in serum of animals and humans. It is understood that the prodrugs of JO-4A useful in the claimed method yield JO-4A and are active in terms of inhibiting the growth of HIV or HSV.

The term "agonists" as used herein refers to substances that elicit the same response (i.e. inhibiting the growth of HIV or HSV in subjects in need of such treatment) as the compounds indicated in formula IV. Agonists of the compounds of formula IV include, but are not restricted to the prodrugs of JO-4A, which prodrugs are illustrated in formula IV.

Methods for determining or screening modified forms of the hypoestoxide compounds i.e. prodrugs and/or agonists of the claimed compounds, for their ability to inhibit the growth of HIV or HSV in subjects in need of such treatment are well known in the art.

(Belshe, R.B., ed., Textbook of Human Virology, 2nded.,1991).

The method of the present invention is directed to antiviral therapy, i.e. inhibiting the growth of lentiviruses or *Herpetoviridae* viruses in subjects using the compounds of formula IV, and in particular, the compounds of formula II (JO-4A). The method of the present invention involves administering to a subject in need of such treatment a therapeutically effective amount of at least one hypoestoxide compound of formulas IV. Preferred hypoestoxides for use in the method are JO-4 (formula I) and JO-4A (formula II). Another aspect of the method involves treating a subject to alleviate pathological effects of the growth of lentiviruses or *Herpetoviridae* viruses, such as HIV or HSV, respectively, in the subject. Pathological effects of HIV and HSV are well known in the art, and are described herein. It is also well understood that inhibiting the growth of HIV or HSV in a subject alleviates the pathology associated with these infections, as is well documented in the literature (Belshe, R.B., ed., Textbook of Human Virology, 2nd ed., 1991).

An embodiment of the method involves associating compounds of formulas IV with a pharmaceutical carrier or diluent for administration to an subject.

As detailed in the Examples below, administering JO-4A to HIV-1 infected PBMCs *in vitro* inhibited the growth of HIV-1, as illustrated in Figures 15A and 15B. At the same dosage level, JO-4A was not toxic to PBMCs (Figure 15B). The growth-inhibiting effect of JO-4A on HSV-2 is illustrated in Figure 16C.

For the above-mentioned use in subjects infected with lentivirus or *Herpetoviridae* viruses, for example, HIV or HSV, respectively, the therapeutic effective amount or dosage will, of course, vary depending on the compound employed, mode of administration and treatment desired. However, in general, satisfactory results would be obtained when administered orally or intravenously at a daily dosage of from about 0.001 mg to about 1000 mg per kg animal body weight, conveniently given in divided doses 1 to 4 times a day or in sustained release form. If administered by injection, in general, satisfactory results would be obtained when administered at a daily dosage of from about 0.001 mg to about 200 mg per kg animal body weight, preferably in the range of from about 50 mg to about 200 mg per kg

conveniently given in divided doses 1 to 4 times a day or in sustained release form. For the larger mammals, the total daily dosage would be in the range of from about 0.00a to about 200 mg, and dosage forms suitable for oral administration comprise from about 0.001 mg to about 1000 mg of the compound admixed or in association with a solid or liquid pharmaceutical carrier or diluent. Methods are well known in the art for determining therapeutically effective amounts of the compounds used in the method of the invention. Such methods involve analysis of the pharmaceutical/ pharmacokinetic parameters in antiviral therapy, i.e for inhibiting the growth of lentiviruses or *Herpetoviridae* viruses, for example, but not restricted to, HIV or HSV in subjects (Elion, G.B., Acyclovir: Discovery, Mechanism of Action, and Selectivity, J. Med. Virol. Suppl. 1:2-6, 1997; Wagstaff, A.J., et al., Drugs 1994, 47( 1 ):153-205).

The method of the present invention includes administering a pharmaceutical composition comprising an effective amount of one or more of the compounds of formula IV. Preferred embodiments are (JO-4A in pure form or as a pharmaceutically acceptable crude concentrate in association with a pharmaceutical carrier or diluent for HIV or HSV-2 infection; and JO-4 for HSV-1 or HSV-2 infection. Such compositions conveniently contain less than 1% by weight, and preferably about 0.2% by weight, of the compounds of formula I and may be prepared by conventional techniques to be in conventional forms, for example, capsules, tablets, suppositories, dispersible powders, syrups, elixirs, suspensions or solutions for enteral or parenteral administration. Suitable pharmaceutical diluents or carriers include, for example, water, alcohols, natural or hardened oils and waxes, calcium and sodium carbonates, calcium phosphate, kaolin, talc and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan mono-oleate, granulating and disintegrating agents such as starch and alginic acid, binding agents such as starch, gelatin and acacia and lubricating agents such as magnesium stearate, stearic acid and talc, in order to provide an elegant and palatable pharmaceutical preparation. Compositions in tablet form may be coated by conventional techniques to delay disintegration of the tablet and absorption of the active ingredient in the gastrointestinal tract and thereby provide sustained action over a long period. Other compounds and methods known in the art for delaying disintegration or for timed-delayed or time-measured delivery of the active ingredients also find use in formulating the active ingredients for use in the methods of the invention. For example, the compounds of formula IV may also be combined with liposomes or other delayed-release carrier means to protect the compounds from degradation until they reach their targets and/or facilitate movement of the compounds across tissue barriers.

The preferred compositions from the standpoint of ease of administration arc solid compositions, particularly solid-filled gelatin capsules or tablets.

It is also to be understood that a further embodiment of the method of the invention involves combining one or more agents in a variety of protocols, including prophylaxis, with the method of the invention for administering to subjects in need of treatment for inhibiting the growth of lentiviruses or *Herpetoviridae* viruses or for alleviating the pathological effects of the growth of those viruses in a subject pharmaceutical compositions comprising compounds of formulas IV. Combination protocols and methods for determining their efficacy, including therapeutic drug monitoring, are well known in the art (Belshe, R.B., ed., Textbook of Human Virology, 2nd ed., 1991). Examples of antiviral agents and other agents useful in therapy for inhibiting the growth of HIV or HSV which may be combined with administering the compounds of formulas IV in the method of the invention include, but are not limited to famciclovir, aciclovir, valaciclovir, sorivudine (BV-arall), BW882C87, ganciclovir, brivudine, cidofovir (HPMPC), lobucavir, ISIS-2922, saqunavir, ritonavir, indinavir, nelfinavir (protease inhibitors); interferon-$\alpha/\beta$, azidothymidine (AZT, Retrovir, Zidovudine), dideoxycytidine (DDC), dideoxyadenosine, dideoxyinosine (DDI), ribavirin, peptide T, soluble recombinant CD4 receptor. Methods are well known in the art for determining therapeutically effective amounts of the compounds of formulas IV and agents selected from the group of anti-viral agents or agents useful in anti-viral therapy in association with the compounds of formulas IV in pharmaceutical compositions in the method of the invention.

## EXAMPLES

The following materials and methods were employed in the non-limiting Examples set out below.

### IMMUNOSUPPRESSION

**Blood donors:** Heparinized blood (30 cc) was obtained by venipuncture from volunteers. Two donors of different genetic backgrounds are usually required to initiate a mixed leukocyte culture (MLC).

**Culture medium:** RPMI-1640 medium (Fisher Scientific Co., Santa Clara, CA) was supplemented with 20% heat-inactivated human AB serum and 1% penicillin/streptomycin mixture.

**Isolation of mononuclear leukocytes:** Mononuclear leukocytes were isolated from the blood by layering appropriate diluted blood in Hanks-balanced salt solution (H-BSS) on ficoll-hypaque gradient followed by centrifugation. The recovered cells were washed three times in culture medium, and viability was determined by trypan blue dye exclusion method. Cell concentration was adjusted to 2 x $10^6$/ml in complete culture medium.

**Mixed leukocyte reaction (MLR)**

The MLR is an assay recognized by those skilled in the art as an *in vitro* predictor of *in vivo* immunosuppressant activity. The MLR is an *in vitro* model of T-cell recognition of foreign major histocompatibility complex (MHC) gene products and was used as a predictive test of cell-mediated graft rejection (Abbas, A.K., et al., eds, 1994, 2nd ed. Cellular and Molecular Immunology, W.B. Saunders Co., USA). Graft rejection is carried out by T cells that recognize MHC molecules and destroy the graft. This process can be studied *in vivo,* but in-depth analysis, especially in humans, required the development of tests such as the MLR as an *in vitro* correlate of graft rejection. (Janeway, Chas. and Travers, Paul, Immunobiology, 2nd Ed., Garland Publishing, Inc, New York (1996)). It is well known in the art that the MLR is induced by co-culturing mononuclear leukocytes from one individual with mononuclear leukocytes derived from another individual. If there are differences in the alleles of the MHC genes between the two individuals, a large proportion of the mononuclear cells will proliferate over a period of 4-7 days, and is referred to as allogeneic MLR. The level of proliferative response is measured by incorporation of $^3$H-thymidine into DNA during cell replication. Because the cells from each donor react and proliferate against the other, the resultant response is known as a "two-way MLR". JO-4 was tested in the MLR for its ability to "immunosuppress".

**"Two-way MLR" set up in microtiter plates**

Mononuclear cells (at 2 x $10^6$/ml) of each donor were first mixed thoroughly together in a 50 ml tube at a 1:1 ratio with volume. The mixture was dispensed into wells of a 96-well U-bottom microtiter plate in volumes of 100 μl per well. Compounds or suspected immunosuppressive agents were added in culture medium in volumes of 100 μl per well at varying concentrations of 1.0, 0.5, or 0.25 μg/ml μ, respectively. 100 μl of culture medium was added to control wells without drug. Cultures were maintained in a humid incubator at 37°C in an atmosphere of 5% $CO_2$ for 5 days. At 4 hours before harvest, the cultures in each well were pulsed with 1 μCi of tritiated thymidine (specific activity 719.5 mCi/mg; Dupont, Wilmington, DE). Cells were harvested onto glass fiber filters (Packard, Downers Grove, IL) with a 96-well automatic cell harvester (TOMTEC, Hamden, CT) and were counted directly on a Matrix 9600 beta counter (Packard). The results are shown in Figure 1.

Data were expressed as percent inhibition (% Inhibition) according to the following formula:

$$\% \text{ Inhibition} = 100 - \frac{\text{(Counts per minute (CPM) for wells with drug x 100)}}{\text{CPM for wells without drug}}$$

**Generation of MHC-restricted Cytolytic CD8+T-lymphocytes in "One-way Human MLR" Cultures**

The "one-way MLR" was set up in tissue culture flasks with mononuclear cells from two different donors as in the "two-way MLR" described above. However, in "one-way MLR", one of the two mononuclear leukocyte populations was rendered incapable of proliferation by treatment with mitomycin C, an antimitotic drug, prior to culture. In this "one-way MLR", the treated cells served exclusively as <u>stimulators</u> and the untreated cells, still capable of proliferation, served as the <u>responders</u>. The responder cells, during a 5-7 day culture period, expressed the CD8+ phenotype but not the CD4+. These CD8+ cells served exclusively as cytolytic T lymphocytes (CTLs) which lyse target cells from the same individual as the original stimulator cell population. In transplantation, these MHC-restricted cytotoxic CD8+ T-cells lyse MHC disparate donor tissue targets, leading to graft rejection (Abbas, A.K., eds., 1994, 2nd ed., Cellular and Molecular Immunology, W.B. Saunders Co., USA; Imagawa, D.K., et al., in Aggarwal, B.B., et al., eds., 1995, Human Cytokines: Their role in Disease and Therapy, Blackwell Science, Inc., Cambridge, MA). The results are shown in Figure 2.

**"One-way" MLR Set up in Tissue Culture Flasks**

Mononuclear cells of one donor were first treated with mitomycin C (Sigma Chem. Co., St. Louis, MO) (50 μg/50 x $10^6$ cells) in 15 mL tubes wrapped in aluminum foil and incubated in a 37°C water bath for 1 hr. At the end of incubation, cells were washed 3 times with tissue culture medium and adjusted to 2 x $10^6$/mL and served as stimulators. Mononuclear cells from another donor which have not been treated with mitomyicin C adjusted to 2 x $10^6$/mL and used as responders. Responders and stimulators were mixed at a 1:1 ratio with volume and incubated in tissue culture flasks for 5-7 days in the presence of either 1.0 μg/mL JO-4 or medium only. Some of the stimulator cells which were not mitomycin C-treated were activated with PHA and followed by culture in IL-2-conditioned media for use as targets at the end of 5 or 7 days in the CTL assay.

**Inhibitory Effect of JO-4 on the induction of Pro-inflammatory cytokine synthesis**

Human peripheral blood mononuclear cells (PBMC) were cultured with B-cell mitogen LPS (Gibco BRL, Grand Island, NY) 3 μg/mL) at 2 x $10^6$/mL in 1.0 mL volumes in 24-well plates in the presence of either 1 μg/mL JO-4 or medium for 48 hr. In a 37°C incubator with 5% $CO_2$. Culture supernatants were collected at the end of incubation period and were assayed by enzyme immunoassay for the presence of the cytokines: interleukin-1β,-6 and TNF-α. Results are expressed as inhibition of cytokine production. The results are shown in Figure 3.

**Inhibitory Effect of JO-4A on human MLR**

Two-way MLR was set up in the absence or presence of varying concentrations of JO-4A (0.1, 1.0, 10, and 100 μM). The results are depicted in Figure 4.

**Inhibitory Effect of JO-4 on LPS-induced murine lymphocyte proliferation *in vitro***

LPS is a B-cell mitogen in mice and it is also known to induce pro-inflammatory cytokines such as TNF-α, IL-6 and IL-1β in both murine and human mononuclear cells (Cunningham, A.J., ed; Understanding Immunology 1978; Academic Press, Inc., New York, USA). Murine lymphocytes were stimulated with LPS *in vitro* in the absence or presence of varying concentrations of JO-4 (1.5, 2.5, 5.0, 10.0, and 50.0 μg/mL). The results are shown in Figure 5.

**Inhibitory Effect of JO-4 on PHA-induced proliferation of human peripheral blood mononuclear cells *in vitro***

Human PBMCs were cultured for 4 days in the absence or presence of varying concentrations of JO-4 (1.25, 2.5, 5.0, 10.0 and 50.0 μg/mL). The results are shown in Figure 6. PHA is a potent T-cell mitogen and stimulates T-cells to produce IL-2.

**Lack of Toxicity of JO-4 on human PBMC**

In order to determine if JO-4 was toxic on human cells *in vitro,* human PBMCs were cultured in the absence or presence of varying concentrations of JO-4 (1.25, 2.5, and 5 μg/mL) for different days (2, 5 and 7 days). At the end of each incubation period, viability of cells was determined by trypan blue dye exclusion method. The results are shown in figure 7.

<u>COMPOUNDS OF THE INVENTION</u>

The compounds tested in the method of the invention included JO-4A (formula II) and JO-4 (formula I) which is an ester of JO-4A.

COMPOUND PREPARATION

<u>Preparation of JO-4A</u>. JO-4 crystals (82 mg, 0.22mmol) were dissolved in a mixture of methanol (3 mL) and dioxane (3 mL) with warming and then cooled to room temperature. Fresh sodium methoxide powder was added to "pH 10". The mixture was stirred at room temperature overnight and the clear, orange-yellow reaction mixture was neutralized with Dowex -50 H+ resin, filtered and evaporated in vacuo to yield a pale yellow syrup which slowly crystallized in the freezer overnight. Yield 65 mg, 90%. <u>**Esters of JO-4.**</u> As shown in formula IV, the compounds of the invention comprise esters of JO-4A, but excluding hypoestoxide (JO-4), which was disclosed in *Heterocycles* 20: 212502128 (1983) and in *Z. Naturoorsch* 37c:558-561 (1982). It should be noted, however, that the method of the invention for inducing immunosuppression involves administering the compounds of formula II, which includes hypoestoxide.

Accordingly, one aspect of the invention is directed to compounds of formula IV

IV

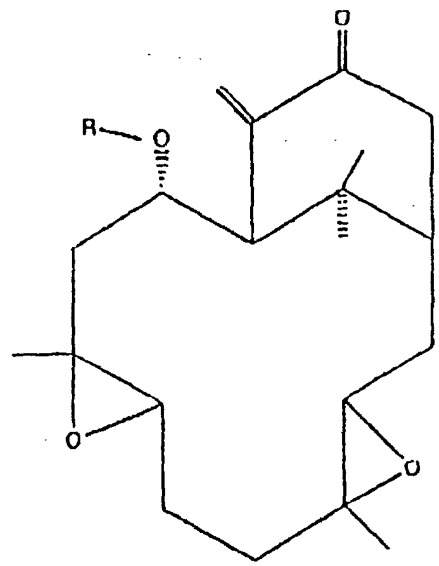

in which

R is
H, $PO_3^{=}$, alkyl of 1 to 12 carbon atoms substituted or unsubstituted, straight chain or branched, 0 to 6 double bonds, $(CH_2)_n$morpholine where n=1-4, morpholinomethylphenyl, orthoaminophenyl, orthohydroxyphenyl, $(CH_2)_nCOOR_2$ where n =1-4
where $R_2$ is H, an alkalai metal salt, an alkaline earth metal salt, $NH_4^+$, $N^+(R_3)_4$ where $R_3$ is independently selected from the group consisting of H and alkyl of 1 to 4 carbon atoms,

$COR_1$ wherein $R_1$ is selected from the group consisting of H, $(CH_2)_nCH_3$, where n=1-6, $(CH_2)_nCOOR_2$ where n=1-4 and $R_2$ is previously defined, $(CH_2)_nN^+(R_3)_4$ wherein n=1-4, and $(CH_2)_nSO_3^-$ where N=1-4, and pharmaceutically acceptable salts thereof. A related aspect of the invention are pharmaceutical compositions comprising a therapeutically effective immunosuppressant amount of the compounds of formula IV.

The methods of the invention for inducing immunosuppression in animals which comprise administering to an animal in need of such treatment a pharmaceutical composition comprising a therapeutically effective amount of the compounds of formula IV, and pharmaceutically acceptable salts thereof.

**Isolation of JO-4 from *Hypoestes rosea*.**

The general procedure for isolation of pure JO-4 (formula I) from dried *Hypoestes rosea* plant material involved solid/liquid extraction using boiling hexanes in a large Soxhlet apparatus. *Hypoestes rosea* is a shrub of the family Acantheceae. (Okugun, J.I. et al., Z. Naturforsch 37c:558-561 (1982)) The crude extract obtained from the hexanes upon evaporation was subjected to flash silica gel column chromatography using a step gradient solvent system beginning with petroleum ether (30-60 bp) and stepping to 5% ethyl acetate, then to 10 % and then 20%. At 30% ethyl acetate JO-4 was eluted from the column. The appropriate fractions were combined and concentrated to dryness, and petroleum ether or hexanes was added to obtain crystalline JO-4. One such procedure provided 240 mg pure JO-4 from 10 g crude extract from leaves.

Important notes: The crude extract was first dissolved in a minimum of ethyl acetate and absorbed onto silica gel and evaporated to a dry powder before loading onto the column, prepacked in petroleum ether. Extraction of specific parts of the plant indicated that the leaves were the structures that contained the majority of the JO-4 as opposed to the stems.

**Results**

As indicated above, the compounds of formula IV were found to have unexpected effectiveness as immunosuppressive and anti-inflammatory agents as shown by their effects on cellular immunity as indicated in standard *in vitro* and *in vivo* tests predictive of a compound's immunosuppressant activity *in vivo* in humans or other animals.

The compounds of formula IV and II, in particular JO-4A and JO-4 as demonstrated herein, immunosuppress formation of, or proliferation or function of immunocytes or lymphocytes, and are therefore useful in suppressing the rejection of transplanted tissues, e.g. organ transplants such as skin, bone marrow, kidney, heart and lung; and useful in preventing graft-versus-host disease (GVHD) and T-cell-mediated autoimmune diseases such as multiple sclerosis (MS), autoimmune thyroiditis, autoimmune myocarditis, systemic lupus erythematosus (SLD), rheumatoid arthritis (RA), Alzheimer's disease (AD), diabetes and glomerulonephritis.

Because the compounds of the invention suppressed the synthesis of pro-inflammatory cytokines (IL-1$\beta$, TNF-$\alpha$, IL-6), the compounds find use for suppressing allergic reactions, asthma, contact dermatitis and skin disease such as psoriasis.

The immunosuppressant effects of the compounds of the invention were determined in the tests described above, the results of which are set forth below. It was found that the immunosuppressant medicinal activity of the claimed compounds in the *in vitro* and mouse *in vivo* tests formed the basis for the inventors' conclusions that the claimed compounds and the pharmaceutical compositions comprising them have *in vivo* efficacy in the inhibition of the immune system in an animal or human host.

**Figure 1** is representative of results from several experiments using different pairs of donors for each experiment. The results demonstrated that JO-4 inhibited the two-way MLR.

**Figure 2** shows the inhibitory effect of JO-4 on MHC class I-restricted cytotoxic T lymphocyte-mediated lysis of MHC-disparate stimulator target cells. In tissue rejection reactions, it is known that MHC-restricted cytotoxic CD8+ T-cells lyse MHC-disparate donor tissue, leading to graft rejection (Abbas et al; Cellular and Molecular Immunology, 1994, W.B. Saunders Co., USA; Imagawa D.K., et al. In Aggarwal, B.B., et al; eds. Human cytokines: Their Role in Disease and Therapy, Blackwell Science, inc., Cambridge, MA, 1995). In this experiment, it was demonstrated that JO-4 significantly (82%) inhibited the lytic capability of such cytotoxic T-cells generated from "one-way human MLR" cultures, a finding which revealed the claimed compounds' immunosuppressant activity *in vitro* and expectant *in vivo* efficacy in the inhibition of the immune system in an animal or human host.

**Figure 3** is representative of results from three experiments which demonstrated the inhibitory or immunosuppressant effect of JO-4 on pro-inflammatory cytokine synthesis (IL-1$\alpha$, TNF-$\beta$ and IL-6). These cytokines are known to be involved in inflammation and endotoxin-shock syndrome (Watkins et al; Pain, 1995, 63:289-302: Immune activation: the role of pro-inflammatory cytokines in inflammation, illness responses and pathological pain states).

**Figure 4** shows the inhibitory effect of JO-4A, on human two-way MLR. The $IC_{50}$ of JO-4A appeared to be between 5.0 $\mu$M and 10.0 $\mu$M. At 10.0 $\mu$M, JO-4A induced 64% inhibition of two-way MLR.

**Figure 5** shows the inhibitory effect of JO-4 on LPS-induced proliferation of murine lymphocytes *in vitro*. Proliferation was inhibited by more than 50% at all concentrations tested (1.5 $\mu$g/mL-50 $\mu$g/mL). Because LPS is a potent murine B lymphocyte mitogen, and B lymphocytes are the producers of plasma cells which secrete antibodies (Cunningham, A.J., ed. 1978; Academic Press, Inc; : Understanding Immunology), JO-4 and the compounds of the invention find use for preventing production of auto-antibodies in autoimmune disease, such as but not restricted to systemic lupus erythromatosus, rheumatoid arthritis.

**Figure 6** shows the inhibitory effect of JO-4 on PHA-induced proliferation of human lymphocytes *in vitro*. PHA activation of lymphocytes leads to IL-2 production which is necessary to maintain or initiate an MLR reaction. Thus, by way of explanation but not limitation, the inhibitory effect of JO-4 on PHA-induced lymphocyte proliferation may explain its inhibitory effect on MLR.

**Figure 7** shows that JO-4 was not toxic to lymphocytes at concentrations shown to inhibit lymphocyte proliferation. Thus, the inhibitory effect of JO-4 on lymphocyte proliferation was due to its capacity to inhibit DNA synthesis without toxicity.

**Figure 8** shows that JO-4A inhibited NK activity *in vivo* by intravenous or by oral administration. This finding demonstrated that the compounds of the invention are useful in suppressing graft-versus host disease by suppressing *in vivo* the activity of NK cells in the donor which are important effector cells when transplanted in systemic acute graft-versus-host disease. Accordingly, the compounds of the invention, as well as JO-4, find use in a method for preventing GVHD in a transplant recipient, which method involves administering to a transplant donor a pharmaceutical composition comprising a therapeutically effective amount of said compounds. A therapeutically effective amount of the compound is sufficient to suppress NK cell function in the donor to avoid or prevent or mitigate GVHD when the NK cells are transplanted to a recipient. It is a matter of routine skill in the art to determine optimum dosages and timing of administration of the compounds to donors in order to mitigate, avoid, or prevent GVHD in a transplant recipient.

## ANTICANCER

The following materials and methods were employed in the non-limiting Examples set out below.

**Tumor cells**: Human cervical epitheloid carcinoma (HeLa), human renal clear cell carcinoma (CAKI-1), human malignant melanoma (RPMI-7951) and mouse malignant melanoma (B16-F1) were procured from ATCC (Rockville, MD)

and maintained *in vitro* with MEM medium (Mediatech, Inc., Herndon, VA) supplemented with 10% heat-inactivated fetal bovine serum (American Qualex, San Clemente, CA), 10mM glutamine, 100 U/ml penicillin, and 100 μg/ml streptomycin (Sigma Chemicals, Saint Louis, MO) (complete medium).

**Normal cells**: Normal human cervical ectoepithelial cells (CrEc-Ec), normal human mammary epithelial cells (HMEC) and normal human umbilical vein endothelial cells (HUVEC) were obtained from Clonetics (San Diego, CA) and cultured with the respective recommended tissue culture medium obtained from Clonetics.

**Peripheral Blood Mononuclear cells (PBMC):** Heparinized venous blood was obtained from healthy adult volunteers and PBMC were separated by centrifugation over Ficoll-Hypaque (Pharmacia Biotech, Piscataway, NJ). The cells were washed three times with HBSS (Hanks Balanced Salt Solution) and finally resuspended in complete medium. Cell viability was assessed by trypan blue dye exclusion test and the cell number was adjusted to required concentration.

**Colorimetric MTT assay:** Cytotoxic effects of the hypoestoxide compounds of formulas I and II (JO-4A) were determined *in vitro* by a colorimetric assay (Mosmann, T., J. Immunol. Methods, 65: 55-63, 1983). Briefly, 1000 cells per well (100 μl) in a 96-well flat-bottom plate were cultured either with the culture medium alone or with various concentrations of drug at 37°C, in a 5% $CO_2$-95% air humidified incubator for 72 hours. At the end of the culture, 10 μl of 5 mg/ml sterile solution of 3-(4,5-Dimethylthiazol-2yl)-2,5-diphenyltetrazolium bromide (MTT) (Sigma Chemicals, Saint Louis, MO) in PBS was added per well and the incubation was resumed for an additional 4 hours. Acid-isopropanol (100 μl of 0.04 N HCl in isopropanol) was added to all wells and kept at room temperature for 30 minutes. Mixing with a multichannel pipetter dissolved the dark blue crystals and the absorbance was measured at 545-650 nm using an ELISA plate reader.

**Mice**: Eight-week-old female C57BL/6 (B6) mice were purchased from Charles River Laboratories (Wilmington, MA.

**The measurement of tumor volume in mice:** B16-F1 melanoma cells growing exponentially *in vitro* were harvested by 15 minutes incubation with 0.25% trypsin-EDTA solution (Irvin Scientific, Irvine, CA). The mice were given subcutaneous (s.c.) injections of 50,000 viable cells on the right side (Fidler, I. J. Cancer Research 35:218-224, 1975). Four days after injection, the mice in the experimental group were gavaged orally with various concentrations of JO-4A in 0.2 ml of water and the control group received water alone. The tumors were measured with a microcaliper, and the tumor volume was calculated by the formula: Length x width x height x 0.5236 (Jungwirth, A. et al., Proc. Natl. Acad. Sci. USA, 94:5810-5813, 1997).

**Statistical analysis:** Students' t-test was used to calculate the level of significance.

Accordingly, the method of the invention comprises administering to subjects therapeutically effective amount of at least one hypoestoxide compound having formula IV and pharmaceutically acceptable salts thereof.

Preferred embodiments of the invention include pharmaceutical compositions comprising a therapeutically effective amount of the compound of formula II (JO-4A) for inhibiting the growth of cancerous tumors.

As shown in the Examples below, the compounds of formula IV, and in particular, formula II (JO-4A) were found to have unexpected effectiveness as agents for inhibiting the growth of cancerous tumors as shown by their effects on cancerous tumor cell growth as indicated in standard *in vitro* and *in vivo* tests predictive of a compound's anti-tumor activity *in vivo* in humans or other animals.

The compounds of formula IV, in particular formula II (JO-4A) as demonstrated herein, inhibit the growth, formation of, or proliferation or function of cancerous tumor cells, and are therefore useful in a method for inhibiting the growth of cancerous tumors in subjects in need of anticancer treatment.

The effects of the compounds of formula IV were determined in the tests described above, the results of which are set forth below. It was found that the medicinal activity of the compounds of formula IV for inhibiting the growth of cancerous tumors compounds in the *in vitro* and mouse *in vivo* tests formed the basis for the inventors' conclusions that the claimed compounds and the pharmaceutical compositions comprising them have *in vivo* efficacy in the inhibition of growth of cancerous tumors in an animal or human host.

## EXAMPLE 1

### Cytotoxic Effects of JO-4A on Cancer Cells *in vitro*

Figure 9 is representative of results from several experiments on the cytotoxic effects of JO-4A on various human cancer cells *in vitro*. The results demonstrated that JO-4A had a dose-dependent cytotoxic effect on human cervical epitheloid carcinoma (HeLa) cells. The inhibitory concentration $_{50}$ ($IC_{50}$), i.e., the drug concentration capable of inducing 50% cytotoxicity, was between 10 μM and 12.5 μM.

Similarly, JO-4A had an $IC_{50}$ of between 20 μM and 40 μM on renal carcinoma (CAKI-1) cells and between 10 μM and 12.5 μM on a malignant melanoma, which metastasizes to lymph node (RPMI-7951).

Figure 10 shows the cytotoxic effects of JO-4A on normal human cells. When normal human cervical ectoepithelial cells, a control cell line for human cervical epitheloid carcinoma (HeLa) were used in the colorimetric assay, JO-4A had an $IC_{50}$ of >100 μM. The $IC_{50}$ dose for HeLa (between 10 μM and 12.5 μM) had only 6% cytotoxic effect

on its normal counterpart. Furthermore, JO-4A had no cytotoxic effect on normal human peripheral blood mononuclear cells. $IC_{50}$ concentration of JO-4A for normal human mammary epithelial cells and normal human umbilical vein endothelial cells were similar (between 25 μM and 50 μM).

**Figure 11** is representative of results from several experiments on the cytotoxic effects of JO-4A on mouse malignant melanoma cells (B16-F1) *in vitro*. The results demonstrated that JO-4A had a dose-dependent cytotoxic effect on B16-F1 cells. The $IC_{50}$ concentration was between 6.25 μM and 12.5 μM.

**EXAMPLE 2**

***In vivo* Effect of JO-4A on Melanoma Tumor Growth**

Figure 12 shows the *in vivo* effect of JO-4A on B16-F1 melanoma tumor in C57BL/6 (B6) mice. Oral administration of JO-4A daily in B6 mice from day 4 post tumor implantation had significantly reduced tumor volume with all four concentrations of JO-4A tested (Figure 12A). Furthermore, the oral administration of JO-4A also increased survival of B6 mice in the experimental group. Whereas all the mice (n = 6) in the non-treated group were dead by day 28, 84% of the mice (5/6) treated with 2 mg/Kg JO-4A survived. Furthermore, 67% (4/6) of the group treated with 1 mg/Kg, 60% (3/5) of the group treated with 0.5 mg/Kg and 17% (1/6) of the group treated with 0.1 mg/Kg survived (Figure 12B).

Figure 13 shows a comparison of survival of each mouse in 2 mg/Kg JO-4A-treated group with non-treated group. The group of mice treated with 2 mg/Kg JO-4A survived longer than the non-treated group, in spite of the fact that treatment was stopped on day 20.

EXAMPLE 3

ANTIVIRAL

The following materials and methods were employed in the non-limiting Examples set out below.

**Peripheral Blood Mononuclear cells (PBMCs):** Heparinized venous blood was obtained from healthy adult volunteers and PBMCs were separated by centrifugation over Ficoll-Hypaque (Pharmacia Biotech, Piscataway, NJ). The cells were washed three times with HBSS (Hanks Balanced Salt Solution) and finally resuspended in RPMI-1640 medium (Mediatech, Inc., Herndon, VA) supplemented with 20% fetal bovine serum (American Qualex, San Clemente, CA), 10 mM glutamine, 100U/ml penicillin, and 100 μg/ml streptomycin (Sigma Chemicals, Saint Louis, MO) (complete medium). Cell viability was assessed by trypan blue dye exclusion test and the cell number was adjusted to required concentration.

**Normal African green monkey kidney cells (CV-1):** CV-1 cells from the Hybriwix™ Probe Systems: Herpes Antiviral Susceptibility Test kit (Diagnostic Hybrids, Inc., Athens, OH) were used for toxicity testing of JO-4A by MTT assay.

***In Vitro* HIV-1 Replication Assay:** A standardized peripheral blood mononuclear cell culture assay for the determination of drug susceptibilities of clinical human HIV-1 isolates was utilized (Japour AJ et al. Antimicro Agents Chemother 1993; 37:1095-1101). Peripheral blood mononuclear cells (2 x $10^6$ cells/ml) were stimulated with PHA (5 μg/ml) in complete culture medium at 37°C, in a 5% $CO_2$-95% air-humidified incubator for 72 hours. The 3-day-old PHA-stimulated PBMC was sedimented at 400 g for 10 min at 20-24°C. Supernatant was removed and discarded. The cells were resuspended in complete culture medium supplemented with 3% IL-2. Cell viability was determined with 0.4% trypan blue. Viability was greater than 85%. Cell concentration was adjusted to 4x$10^6$/ml. Cells were dispensed in 50 μl volumes (2x$10^5$/well) into 96 well microtiter plates containing 50 μl complete culture medium. HIV-1 infected cultures were initiated with a patient's HIV-1 isolate (50 μl/well) at a multiplicity of infection (MOI) of 0.05 and 0.1 respectively. JO-4A was added to wells in 50 μl volumes at varying concentrations (0.0001 μM - 0.5 μM). The plates were incubated at 37°C and 5% $CO_2$ in humidified chamber for 7 days. On day 4, half of the spent medium (100 μl) was discarded, and 50 μl of fresh complete medium and 50 μl of fresh JO-4A (varying concentrations as above) were added to wells containing spent JO-4A. 100 μl of fresh medium was added to wells without drug. On day 7, the culture was terminated and the supernatants were tested for HIV p24 antigen by an enzyme immunoassay (EIA) kit according to the recommendations of the manufacturer (Coulter™ HIV p24 Antigen Assay: Immunotech, Inc., Westbrook, Maine).

***In Vitro Herpes simplex* virus (HSV) (Type 1 and 2) Replication Assay:**

The antiviral activity of the drug was determined using the Hybriwix™ Probe Systems (Herpes Antiviral Susceptibility Test kit: Diagnostic Hybrids, Inc., Athens, OH). Briefly, either HSV-1 or HSV-2 isolates (ATTC, Rockville, MD) were pre-adsorbed at 37°C and 5% $CO_2$ in a humidified incubator for 60 minutes on African green monkey Kidney cells in a 24-well flat-bottom plate. Following pre-adsorption, the viral inoculum was removed and the growth medium

containing various concentrations of appropriate drug was placed in appropriate wells. The culture was continued for 48 hours for the amplification of the virus. Quantification of the HSV DNA present in each well was determined by the nucleic acid hybridization method. The cells and/or virus were lysed with DNA wicking agent and the single-stranded DNA was immobilized on a Hybriwix™ filter by a vertical capillary absorption of the wicking agent/DNA solution. The amount of HSV target DNA from each well was determined using radioactive ($I^{125}$)-HSV DNA probe containing genetic sequences, which are homologous to HSV-1 or HSV-2 sequences. The amount of radioactivity present on the Hybriwix™ was dependent upon the amount of HSV DNA present after culture, and was determined in a gamma counter (Packard Instrument Co., Meriden, CT).

**Colorimetric MTT assay:** Cytotoxic effects of drugs were determined *in vitro* by a colorimetric assay (Mosmann, T., J. Immunol. Methods, 65: 55-63, 1983). Briefly, 1000 cells per well (100 µl) in a 96-well flat-bottom plate were cultured either with the culture medium alone or with various concentrations of drug at 37°C, in a 5% $CO_2$-95% air humidified incubator for 72 hours. At the end of the culture, 10 µl of 5 mg/ml sterile solution of 3-(4,5-Dimethylthiazol-2yl)-2,5-diphenyltetrazolium bromide (MTT) (Sigma Chemicals, Saint Louis, MO) in PBS was added per well and the incubation was resumed for an additional 4 hours. Acid-isopropanol (100 µl of 0.04 N HCl in isopropanol) was added to all wells and kept at room temperature for 30 minutes. Mixing with a multichannel pipetter dissolved the dark blue crystals and the absorbance was measured at 545-650 nm using an ELISA plate reader.

## COMPOUNDS USED IN THE METHOD

Hypoestoxide compounds (formula IV) tested in the method of the invention included JO-4A (formula II) and JO-4 (formula I), which is an ester of JO-4A.

Accordingly, the method of the invention comprises administering to subjects a therapeutically effective amount of at least one hypoestoxide compound having formula IV and pharmaceutically acceptable salts thereof. A preferred embodiment of the method of the invention administers pharmaceutical compositions comprising a therapeutically effective amount of the compound of formula II (JO-4A) for inhibiting the growth of HIV and HSV-2. Another preferred embodiment of the method of the invention administers pharmaceutical compositions comprising a therapeutically effective amount of the compound of formula I (JO-4) for inhibiting the growth of HSV-1 or IV and HSV-2.

As shown in the Examples below, the compounds of formulas IV (JO-4) and II (JO-4A) were found to have unexpected effectiveness as agents for inhibiting the growth of, respectively, HSV1 and HSV2, and of HIV and HSV-2 as shown by their effects on viral growth as indicated in standard *in vitro* predictive of a compound's anti-viral activity *in vivo* in humans or other animals.

The compound of formula I (JO-4) as demonstrated herein, inhibited the replication and growth of HSV-1 and HSV-2 in African Green Monkey kidney cells, a screening test well known in the art for predicting anti-HSV-1 or anti-HSV-2 activity in a subject, and from which one of reasonable skill in the art would consider JO-4 useful in a method for inhibiting the growth of HSV-1 or HSV-2 in subjects in need of anti-viral therapy, thereby alleviating the pathological effects of HSV-1 or HSV-2 infection.

The compound of formula II (JO-4A as demonstrated herein, inhibited the replication and growth of HIV in human PBMCs and HSV-2 in African Green Monkey kidney cells, screening tests well known in the art for predicting anti-HIV or anti-HSV-2 activity, respectively, in a subject, and from which one of reasonable skill in the art would consider JO-4A useful in a method for inhibiting the growth of HIV or HSV-2 in subjects in need of anti-viral therapy, thereby alleviating the pathological effects of HIV or HSV-2 infection, respectively.

It was found that the medicinal activity of the compounds of formula IV - in particular JO-4 and JO-4A, for inhibiting the growth of HIV or HSV in the *in vitro* tests reported below formed the basis for the inventors' conclusions that the compounds, and that the pharmaceutical compositions comprising them have *in vivo* efficacy in the inhibition of growth of HIV or HSV in an animal or human host.

## EXAMPLES

### Example 1

### Anti-Viral Effect of JO-4A on HIV Growth in PBMCs

**Figure 15A** is representative of results from several experiments on the inhibitory effects of JO-4A on HIV-1 isolate from a patient. The results demonstrate that JO-4A has the dose dependent inhibitory effect on HIV-1 replication and it was concluded that the inhibitory concentration $_{50}$ ($IC_{50}$), i.e., the drug concentration capable of inhibiting 50%, was between 0.0001 µM and 0.01 µM.

**Figure 15B** shows the toxicity testing results of JO-4A on cultured human PBMC by trypan blue dye-exclusion test. JO-4A has no toxic effect at any of the concentrations tested. Therefore, the inhibitory effect on HIV-1 replication

by JO-4A is not due to cytotoxicity of JO-4A on PBMCs.

**Example 2**

**Anti-Viral Effect of JO-4 on HSV-1 and HSV-2 Growth**

**Figure 16A** shows the antiviral effect of JO-4 on HSV-1 replication. Following two days of culture with various concentrations of JO-4, HSV-1 replication in African green monkey kidney cells was inhibited by 60% with 12.5 μM and 53% with 3.125 μM. Thus, the $IC_{50}$ dose for JO-4 on HSV-1 inhibition was determined to be ~3.125 μM.

**Figure 16B** shows the antiviral effect of JO-4 on HSV-2 replication. After two days of culture with various concentrations of JO-4, HSV-2 replication in African green monkey kidney cells was inhibited by 65% with 12.5 μM and 58% with 3.125 μM. The $IC_{50}$ dose for JO-4 on HSV-1 inhibition was determined to be < 3.125 μM.

**Example 3**

**Toxicity Study of JO-4 and JO-4A on Cultured Cells**

**Figure 17A and 17B** show the results of toxicity testing of JO-4 and JO-4A on normal, uninfected African green monkey kidney cells (CV-1). When CV-1 cells were cultured either in the presence or absence of various concentrations of JO-4 and JO-4A for 72 hours, cytotoxicity of greater than 50% was obtained only at higher concentrations of the compounds (> 50 μM for JO-4; and ~ 25 μM for JO-4A). Therefore, the inhibitory effect of JO-4 and JO-4A on HSV-1 and HSV-2 replication was not due to the cytotoxicity of the respective drugs against CV-1 cells.

## Claims

**1.** A pharmaceutical composition comprising a pharmaceutical carrier or diluent and a compound having the formula:

(IV)

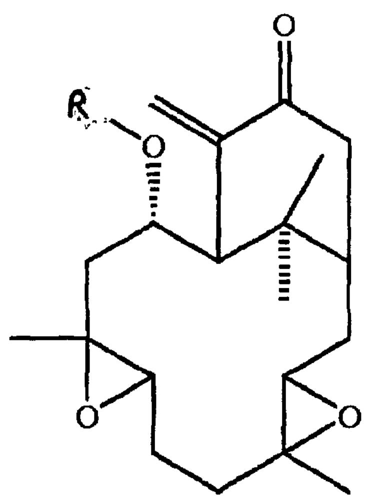

where R is H, $COR_1$, $PO_3^{=}$, alkyl of 1 to 12 carbon atoms substituted or unsubstituted, straight chain or branched, 0 to 6 double bonds, $(CH_2)_n$morpholine where n=1-4, morpholinomethylphenyl, orthoaminophenyl, orthohydroxyphenyl, $(CH_2)_n$ $COOR_2$ where n=1-4

- where $R_2$ is H, an alkali metal salt, an alkaline earth metal salt, $NH_4^+$, $N^+(R_3)_4$;
- where $R_3$ is independently selected from the group consisting of H and alkyl of 1-4 carbon atoms;
- where $R_1$ is selected from the group consisting of H, $(CH_2)_n$CH3, where n=0-6, $(CH_2)_nCOOR_2$ where n=1-4 and $R_2$ is previously defined, $(CH_2)_nN^+(R_3)_4$ wherein n=1-4, and $(CH_2)_nSO_3^-$ where n=1-4;

and pharmaceutically acceptable salts thereof.

**2.** The composition according to claim 1 wherein R is H.

**3.** Use of the compound having the formula (IV) as defined in claim 1 for the preparation of an immunosuppressant.

**4.** Use of the compound having the formula (IV) as defined in claim 1 for the preparation of a pharmaceutical composition for inhibiting growth of cancer cells.

**5.** The use according to claim 4 wherein said cancer cells are selected from the group of cancer cells consisting of cervical carcinoma, metastatic melanoma, renal cell carcinoma, testicular embryonal carcinoma, and colonic carcinoma.

**6.** Use of the compound having the formula (IV) as defined in claim 1 for the preparation of a pharmaceutical composition for inhibiting the growth of lentiviruses or *Herpetoviridae* viruses.

**7.** The use according to claim 6 wherein said lentivirus is selected from the group consisting of HIV-1, HIV-2, and HTLV.

**8.** The use according to claim 6 wherein said *Herpetoviridae* virus is selected from the group consisting of herpes simplex virus, Epstein-Barr virus, cytomegalovirus, and varicella-zoster virus.

**9.** The compound having the formula (IV) as defined in claim 1 wherein $R_1$ is $(CH_2)_nCH_3$ and n=1-6.

**Patentansprüche**

**1.** Pharmazeutische Zusammensetzung, aufweisend einen pharmazeutischen Träger oder Streckmittel und eine Verbindung der Formel:

IV

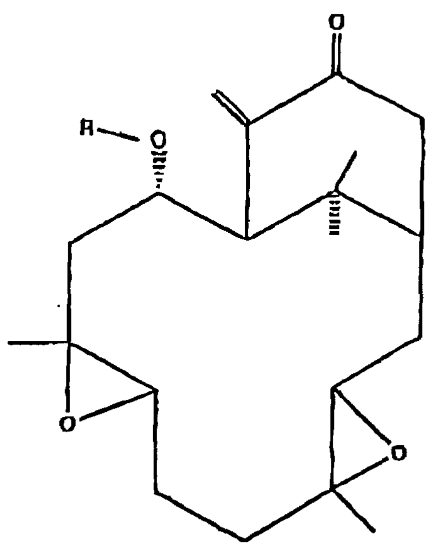

worin R H ist, $COR_1$; $PO_3^{2-}$; ein Alkyl mit 1 bis 12 Kohlenstoffatomen, substituiert oder unsubstituiert, geradkettig oder verzweigt, Null bis 6 Doppelbindungen; $(CH_2)_n$-Morpholin, worin n 1 bis 4 beträgt; Morpholinomethylphenyl; Orthoaminophenyl; Orthohydroxyphenyl; $(CH_2)_nCOOR_2$, worin n 1 bis 4 beträgt;

- worin $R_2$ H ist; ein Alkalimetallsalz; ein Erdalkalimetallsalz; $NH_4^+$; $N^+(R_3)_4$;
- worin $R_3$ unabhängig ausgewählt ist aus der Gruppe, bestehend aus H und Alkyl mit 1 bis 4 Kohlenstoffatomen;
- worin $R_1$ ausgewählt ist aus der Gruppe, bestehend aus H; $(CH_2)_nCH_3$, worin n Null bis 6 beträgt; $(CH_2)_nCOOR_2$, worin n 1 bis 4 beträgt und $R_2$ wie vorstehend festgelegt ist; $(CH_2)_nN^+(R_3)_4$, worin n 1 bis 4 beträgt und $(CH_2)_nSO_3^-$, worin n 1 bis 4 beträgt;

sowie pharmazeutisch zulässige Salze davon.

**2.** Zusammensetzung nach Anspruch 1, worin R H ist.

**3.** Verwendung der Verbindung mit der Formel (IV) nach Anspruch 1 zur Herstellung eines Immunsuppressivums.

**4.** Verwendung der Verbindung mit der Formel (IV) nach Anspruch 1 zur Herstellung einer pharmazeutischen Zusammensetzung zur Hemmung des Wachstums von Krebszellen.

**5.** Verwendung nach Anspruch 4, worin die Krebszellen ausgewählt sind aus der Gruppe, bestehend aus Krebszellen von Zervixkarzinom, metastatischem Melanom, hypemephroidem Karzinom, embryonalem Hodenkarzinom, Dickdarmkarzinom.

**6.** Verwendung der Verbindung mit der Formel (IV) nach Anspruch 1 zur Herstellung einer pharmazeutischen Zusammensetzung zur Hemmung des Wachstums von Lentiviren oder *Herpetoviridae*-Viren.

**7.** Verwendung nach Anspruch 6, worin das Lentivirus ausgewählt ist aus der Gruppe, bestehend aus HIV-1, HIV-2 und HTLV.

**8.** Verwendung nach Anspruch 6, worin das *Herpetoviridae*-Virus ausgewählt ist aus der Gruppe, bestehend aus Herpes-Simplex-Virus, Epstein-Barr Virus, Cytomegalovirus und Varicella-Zoster-Yirus.

**9.** Verbindung mit der Formel (IV) nach Anspruch 1, worin $R_1$ $(CH_2)_nCH_3$ ist und n 1 bis 6 beträgt.

**Revendications**

**1.** Composition pharmaceutique comprenant un véhicule pharmaceutique ou un diluant et un composé répondant à la formule:

IV

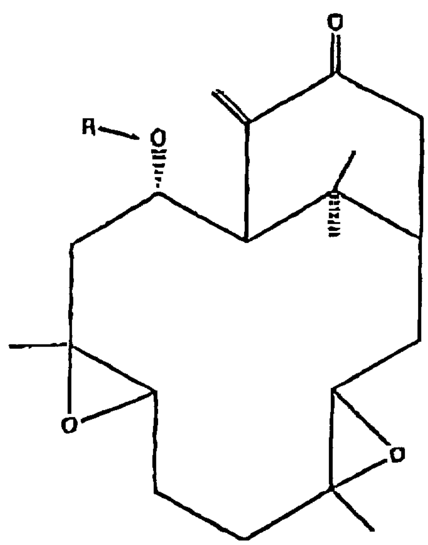

dans laquelle R représente H, $COR_1$, $PO_3^{2-}$, un groupement alkyle à 1 à 12 atomes de carbone, substitué ou non substitué, à chaîne linéaire ou ramifiée et comportant 0 à 6 liaisons doubles, un groupement $(CH_2)_n$-morpholine où n = 1 - 4, (morpholinométhyl)phényle, (orthoamino)phényle, (orthohydroxy)phényle ou $(CH_2)_nCOOR_2$ où n = 1 - 4,

- où $R_2$ représente H, le sel d'un métal alcalin, le sel d'un métal alcalino-terreux, $NH_4^+$ ou $N^+(R_3)_4$;
- où $R_3$ est indépendamment sélectionné parmi le groupe consistant en H et un groupement alkyle à 1 à 4 atomes de carbone;
- où $R_1$ est sélectionné parmi le groupe consistant en H, $(CH_2)_nCH_3$ où n = 0 - 6, $(CH_2)_nCOOR_2$ où n = 1 - 4 et $R_2$ est tel que défini plus haut, $(CH_2)_nN^+(R_3)_4$ où n = 1 - 4 et $(CH_2)_nSO_3^-$ où n = 1 - 4;

et les sels pharmaceutiquement acceptables de ces composés.

**2.** Composition selon la revendication 1, dans laquelle R représente H.

**3.** Utilisation d'un composé répondant à la formule (IV) telle que définie à la revendication 1 dans la préparation d'un immunosuppresseur.

**4.** Utilisation d'un composé répondant à la formule (IV) telle que définie à la revendication 1 dans la préparation d'une composition pharmaceutique pour inhiber la croissance de cellules cancéreuses.

**5.** Utilisation selon la revendication 4, dans laquelle lesdites cellules cancéreuses sont sélectionnées parmi le groupe consistant en un cancer du col de l'utérus, un mélanome métastatique, un carcinome à cellules rénales, un carcinome embryonnaire testiculaire et un cancer du côlon.

**6.** Utilisation d'un composé répondant à la formule (IV) telle que définie à la revendication 1 dans la préparation d'une composition pharmaceutique pour inhiber la croissance des lentivirus ou des virus du genre *Herpetoviridae*.

**7.** Utilisation selon la revendication 6, dans laquelle ledit lentivirus est sélectionné parmi le groupe consistant en le VIH-1, le VIH-2 et l'HTLV.

**8.** Utilisation selon la revendication 6, dans laquelle ledit virus du genre *Herpetoviridae* est sélectionné parmi le groupe consistant en le virus Herpes simplex, le virus d'Epstein-Barr, le cytomégalovirus et l'herpesvirus varicellae.

**9.** Composé répondant à la formule (IV) telle que définie à la revendication 1, dans laquelle $R_1$ représente $(CH_2)_nCH_3$ et n = 1 - 6.

FIG. 1

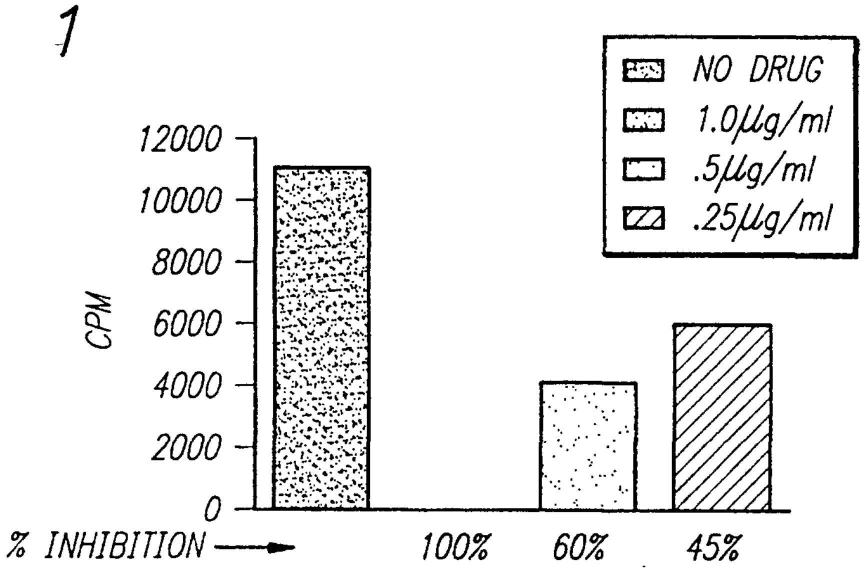
NO DRUG
1.0μg/ml
.5μg/ml
.25μg/ml
12000
10000
8000
6000
4000
2000
0
CPM
% INHIBITION
100%
60%
45%

FIG. 2

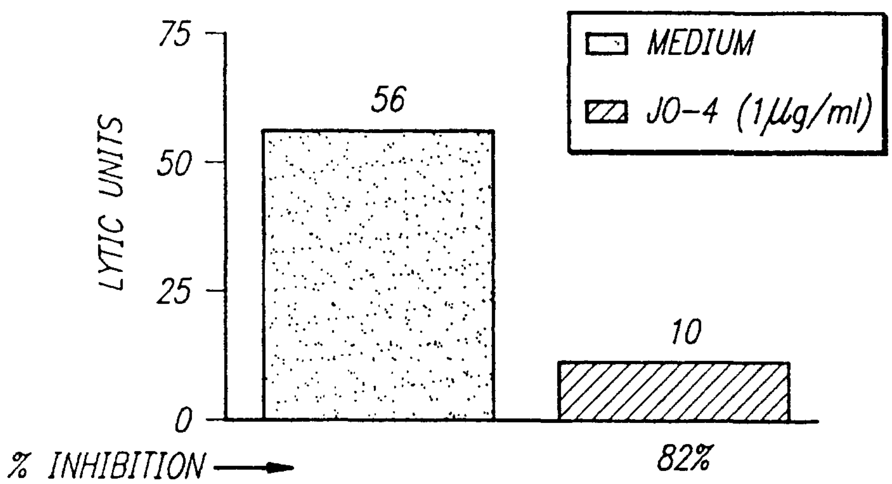
MEDIUM
JO-4 (1μg/ml)
75
50
25
0
LYTIC UNITS
56
10
% INHIBITION
82%

FIG. 3

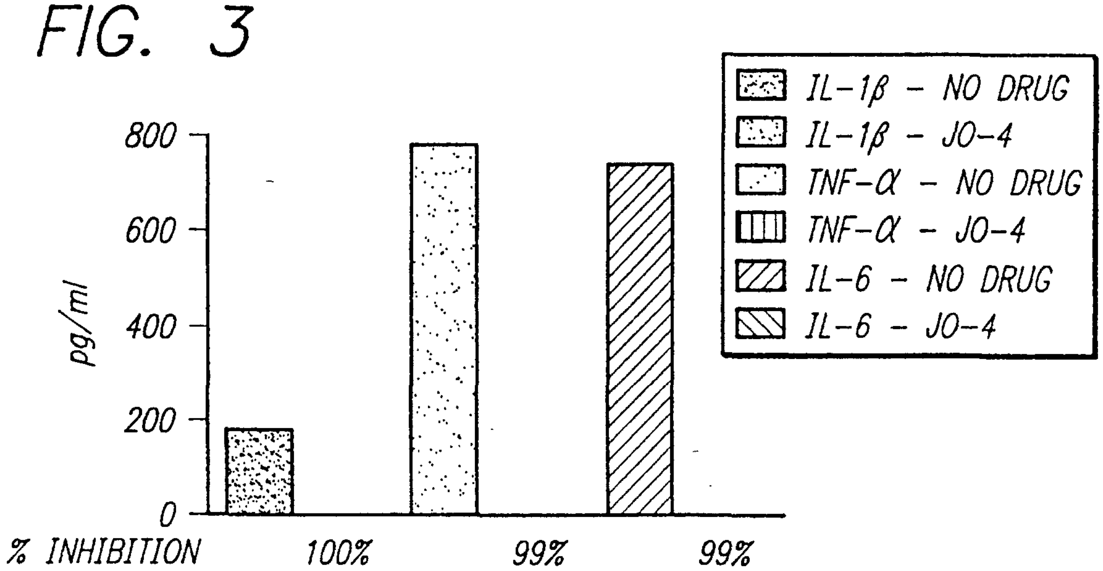
IL-1β - NO DRUG
IL-1β - JO-4
TNF-α - NO DRUG
TNF-α - JO-4
IL-6 - NO DRUG
IL-6 - JO-4
800
600
400
200
0
pg/ml
% INHIBITION
100%
99%
99%

FIG. 4

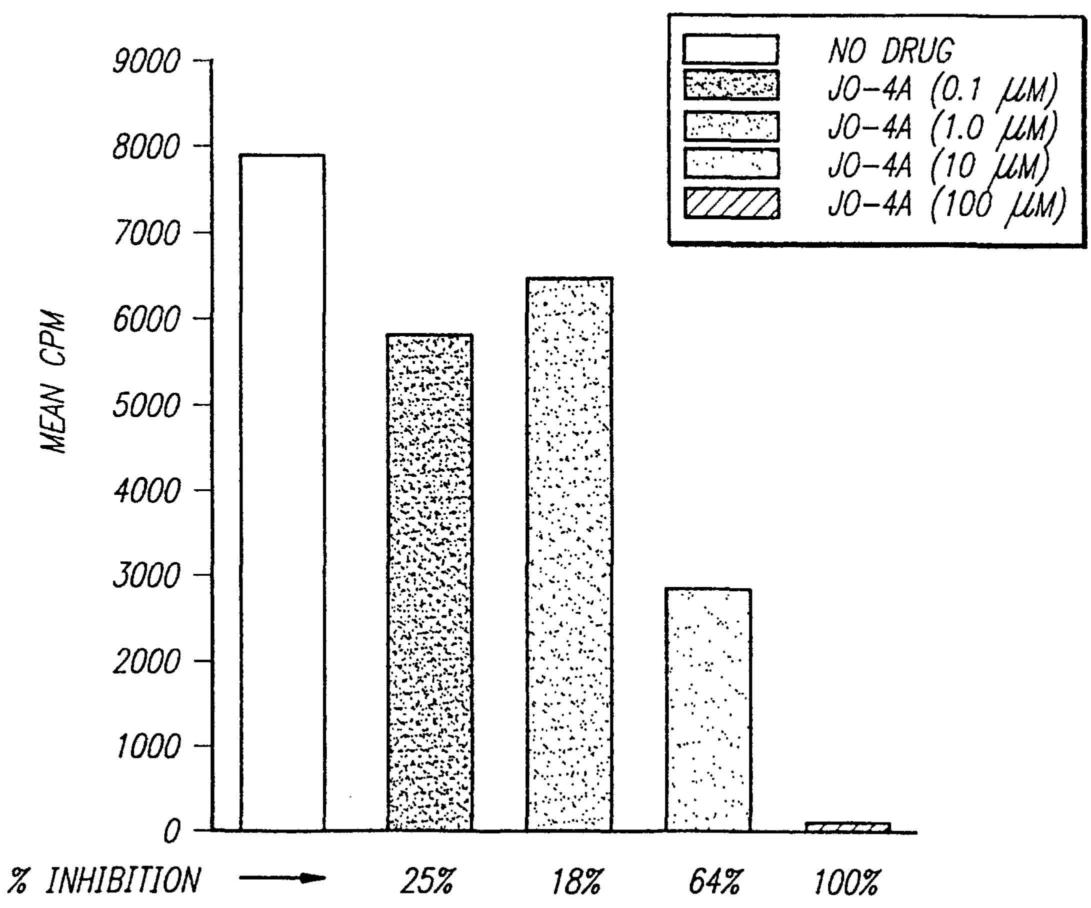
NO DRUG
JO-4A (0.1 μM)
JO-4A (1.0 μM)
JO-4A (10 μM)
JO-4A (100 μM)
9000
8000
7000
6000
5000
4000
3000
2000
1000
0
MEAN CPM
% INHIBITION
25%
18%
64%
100%

FIG. 5

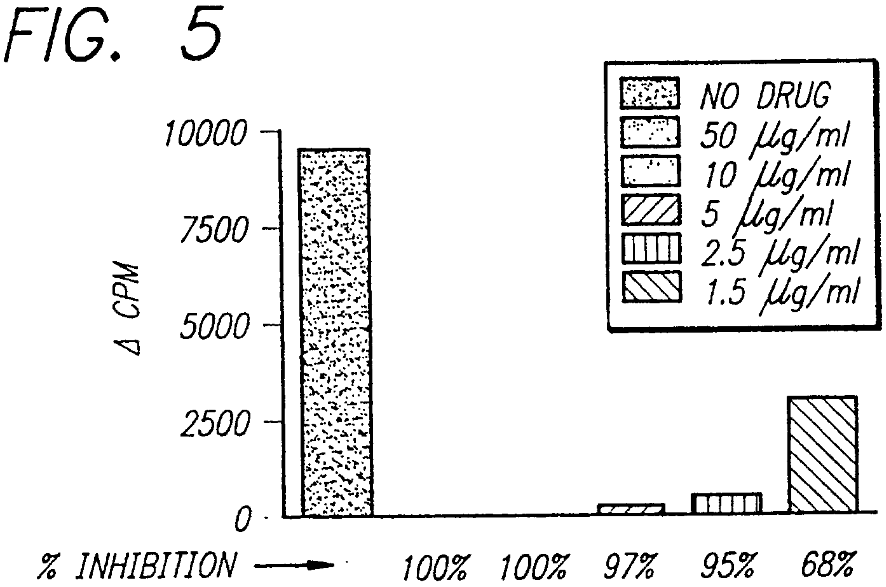
NO DRUG
50 μg/ml
10 μg/ml
5 μg/ml
2.5 μg/ml
1.5 μg/ml
10000
7500
5000
2500
0
Δ CPM
% INHIBITION
100%
100%
97%
95%
68%

FIG. 6

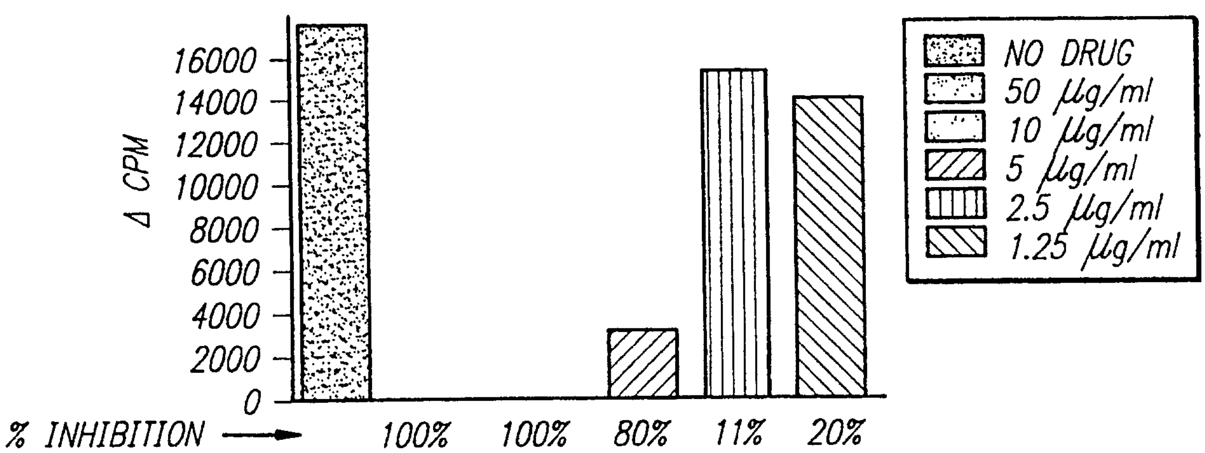
NO DRUG
50 μg/ml
10 μg/ml
5 μg/ml
2.5 μg/ml
1.25 μg/ml
16000
14000
12000
10000
8000
6000
4000
2000
0
Δ CPM
% INHIBITION
100%
100%
80%
11%
20%

FIG. 7

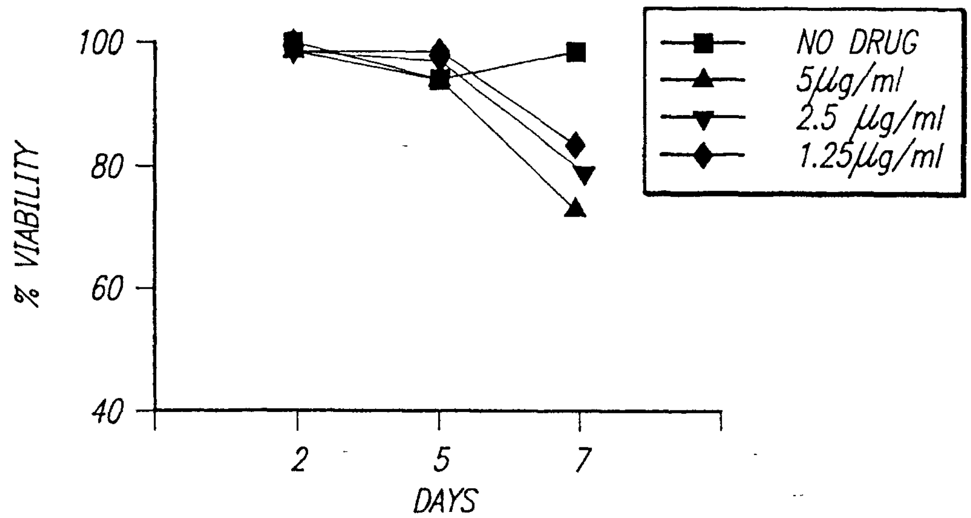
100
80
60
40
% VIABILITY
2
5
7
DAYS
NO DRUG
5μg/ml
2.5 μg/ml
1.25μg/ml

FIG. 11

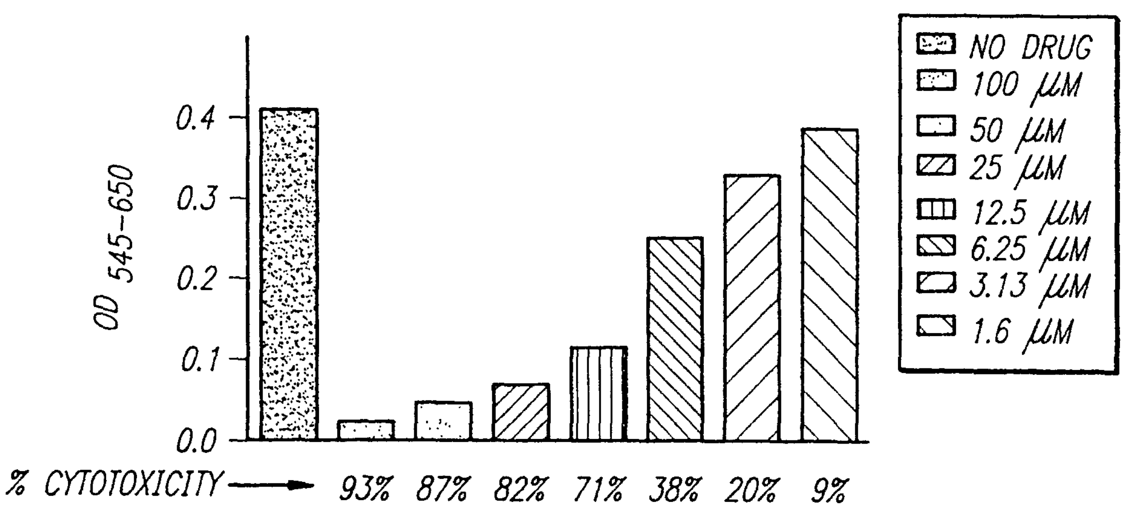
0.4
0.3
0.2
0.1
0.0
OD 545-650
% CYTOTOXICITY
93%
87%
82%
71%
38%
20%
9%
NO DRUG
100 μM
50 μM
25 μM
12.5 μM
6.25 μM
3.13 μM
1.6 μM

FIG. 8A

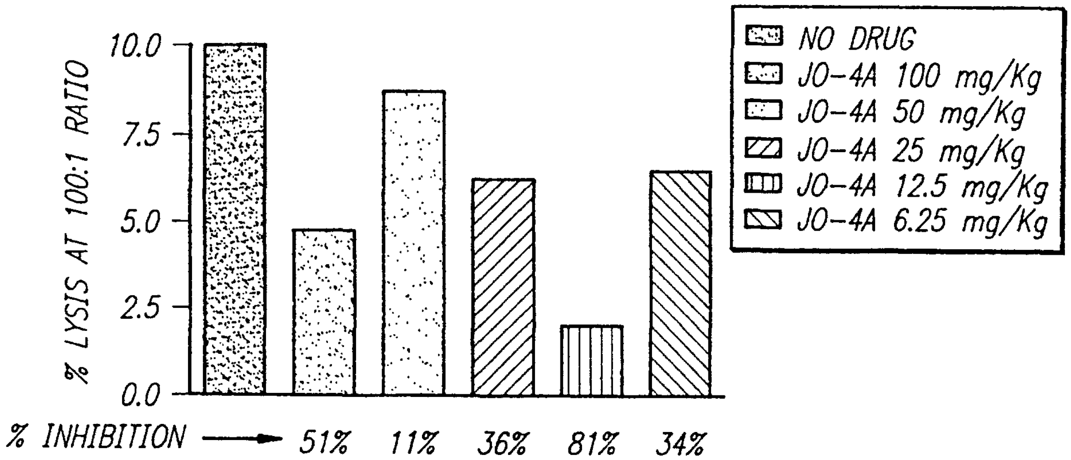
NO DRUG
JO-4A 100 mg/Kg
JO-4A 50 mg/Kg
JO-4A 25 mg/Kg
JO-4A 12.5 mg/Kg
JO-4A 6.25 mg/Kg
% LYSIS AT 100:1 RATIO
10.0
7.5
5.0
2.5
0.0
% INHIBITION
51%
11%
36%
81%
34%

FIG. 8B

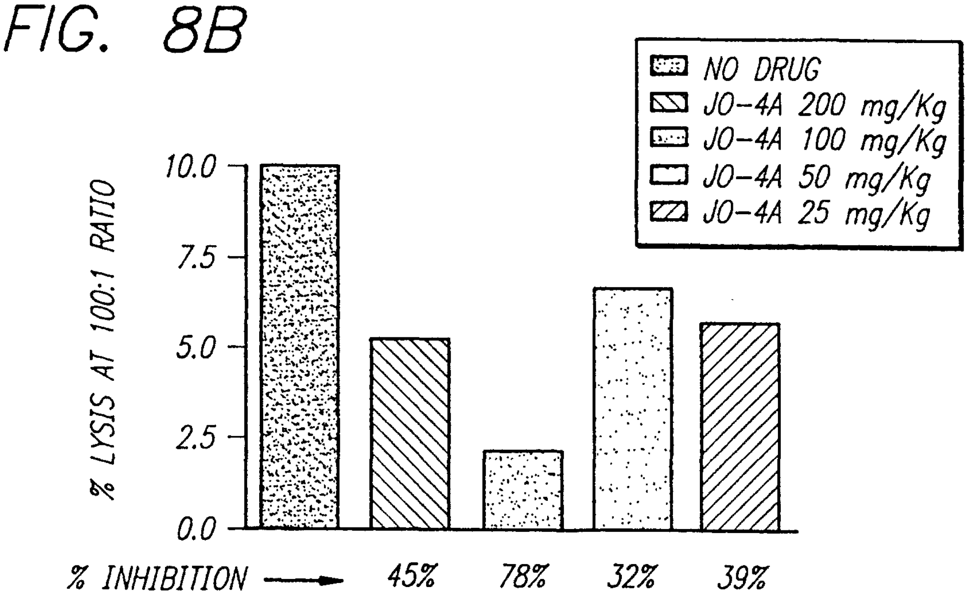
NO DRUG
JO-4A 200 mg/Kg
JO-4A 100 mg/Kg
JO-4A 50 mg/Kg
JO-4A 25 mg/Kg
% LYSIS AT 100:1 RATIO
10.0
7.5
5.0
2.5
0.0
% INHIBITION
45%
78%
32%
39%

FIG. 9

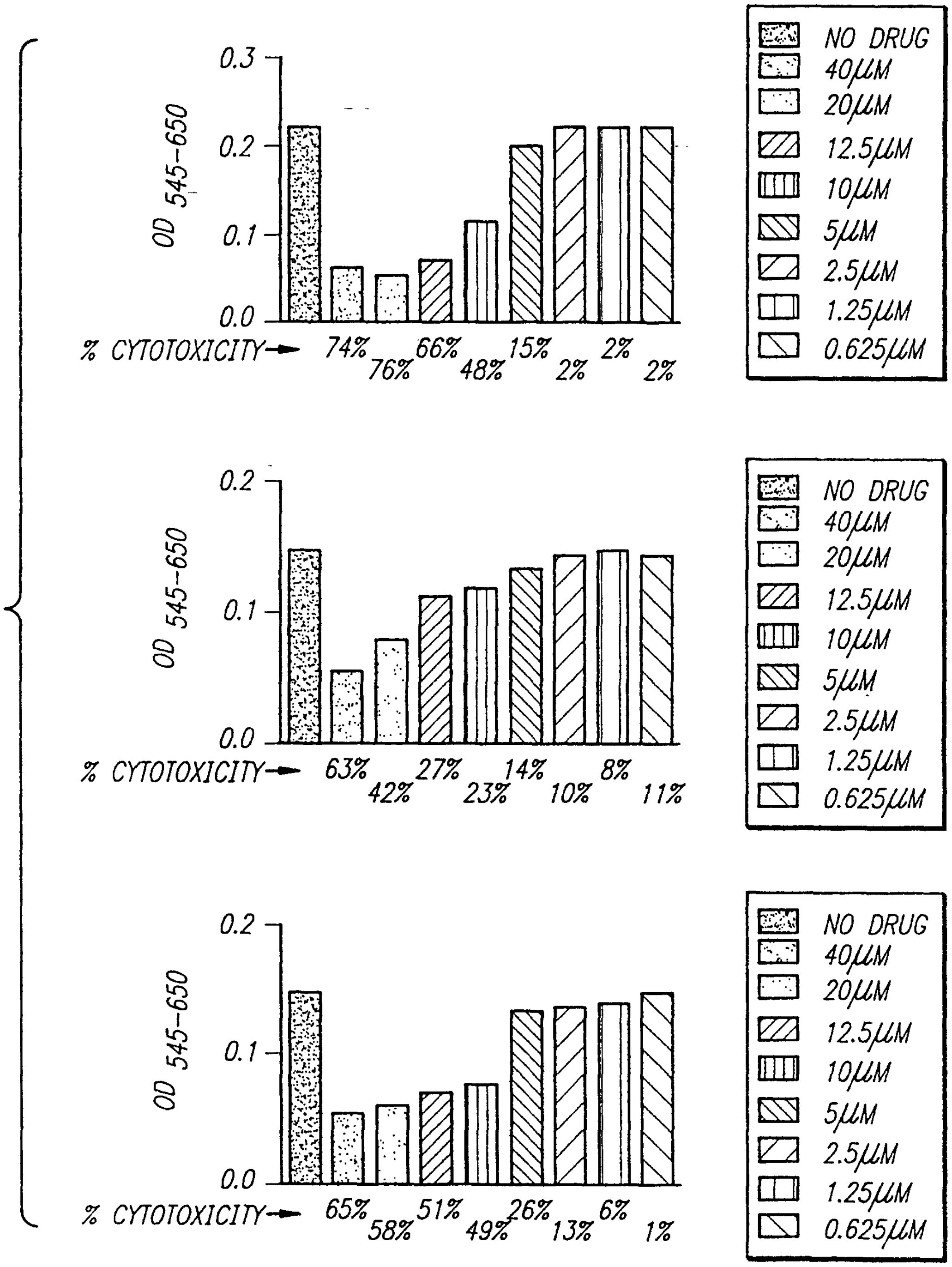
0.3
0.2
0.1
0.0
OD 545-650
% CYTOTOXICITY
74%
76%
66%
48%
15%
2%
2%
2%
NO DRUG
40μM
20μM
12.5μM
10μM
5μM
2.5μM
1.25μM
0.625μM
0.2
0.1
0.0
OD 545-650
% CYTOTOXICITY
63%
42%
27%
23%
14%
10%
8%
11%
NO DRUG
40μM
20μM
12.5μM
10μM
5μM
2.5μM
1.25μM
0.625μM
0.2
0.1
0.0
OD 545-650
% CYTOTOXICITY
65%
58%
51%
49%
26%
13%
6%
1%
NO DRUG
40μM
20μM
12.5μM
10μM
5μM
2.5μM
1.25μM
0.625μM

FIG. 10-1

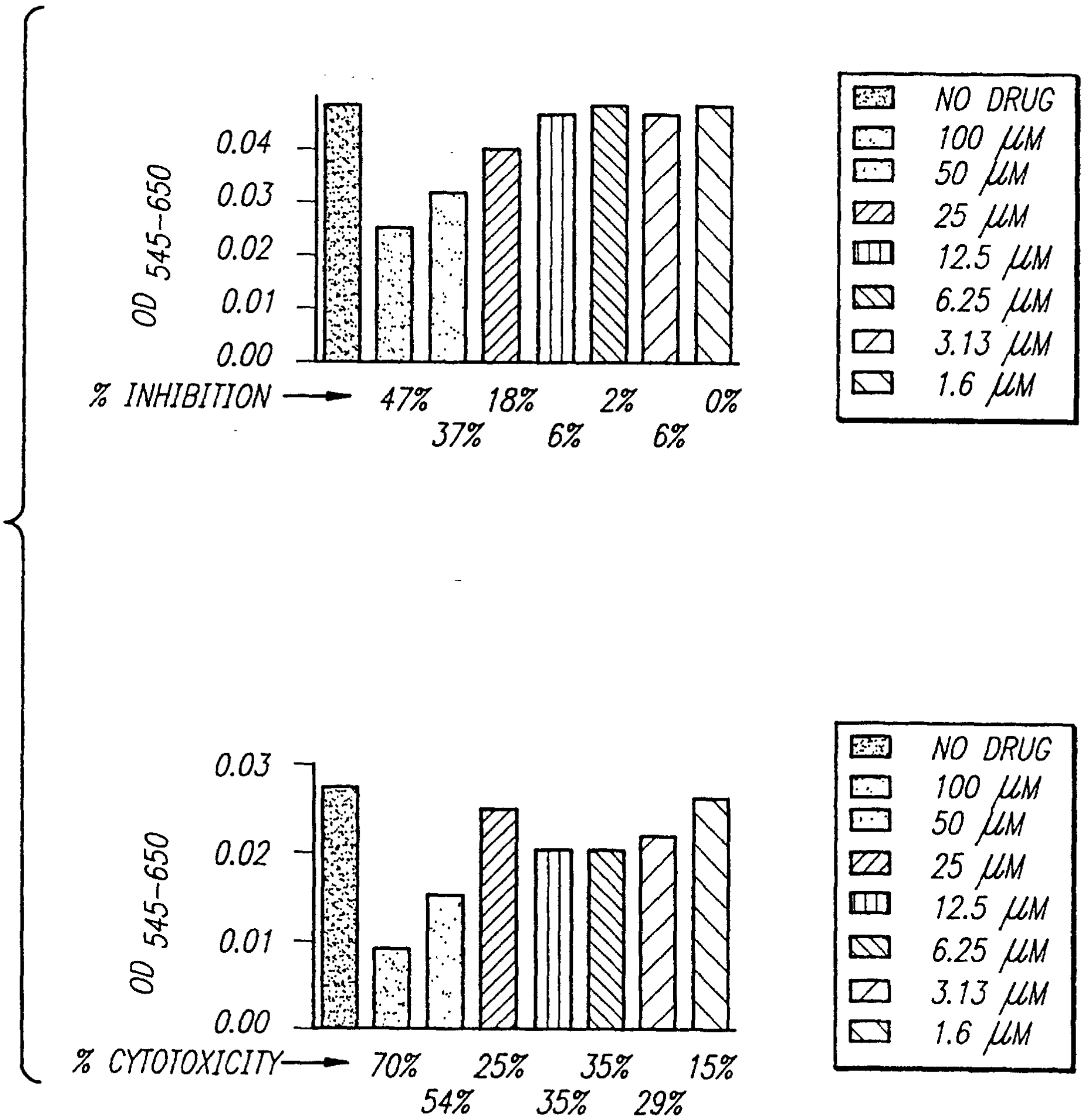
0.04
0.03
0.02
0.01
0.00
OD 545-650
% INHIBITION
47%
37%
18%
6%
2%
6%
0%
NO DRUG
100 μM
50 μM
25 μM
12.5 μM
6.25 μM
3.13 μM
1.6 μM
0.03
0.02
0.01
0.00
OD 545-650
% CYTOTOXICITY
70%
54%
25%
35%
35%
29%
15%
NO DRUG
100 μM
50 μM
25 μM
12.5 μM
6.25 μM
3.13 μM
1.6 μM

FIG. 10-2

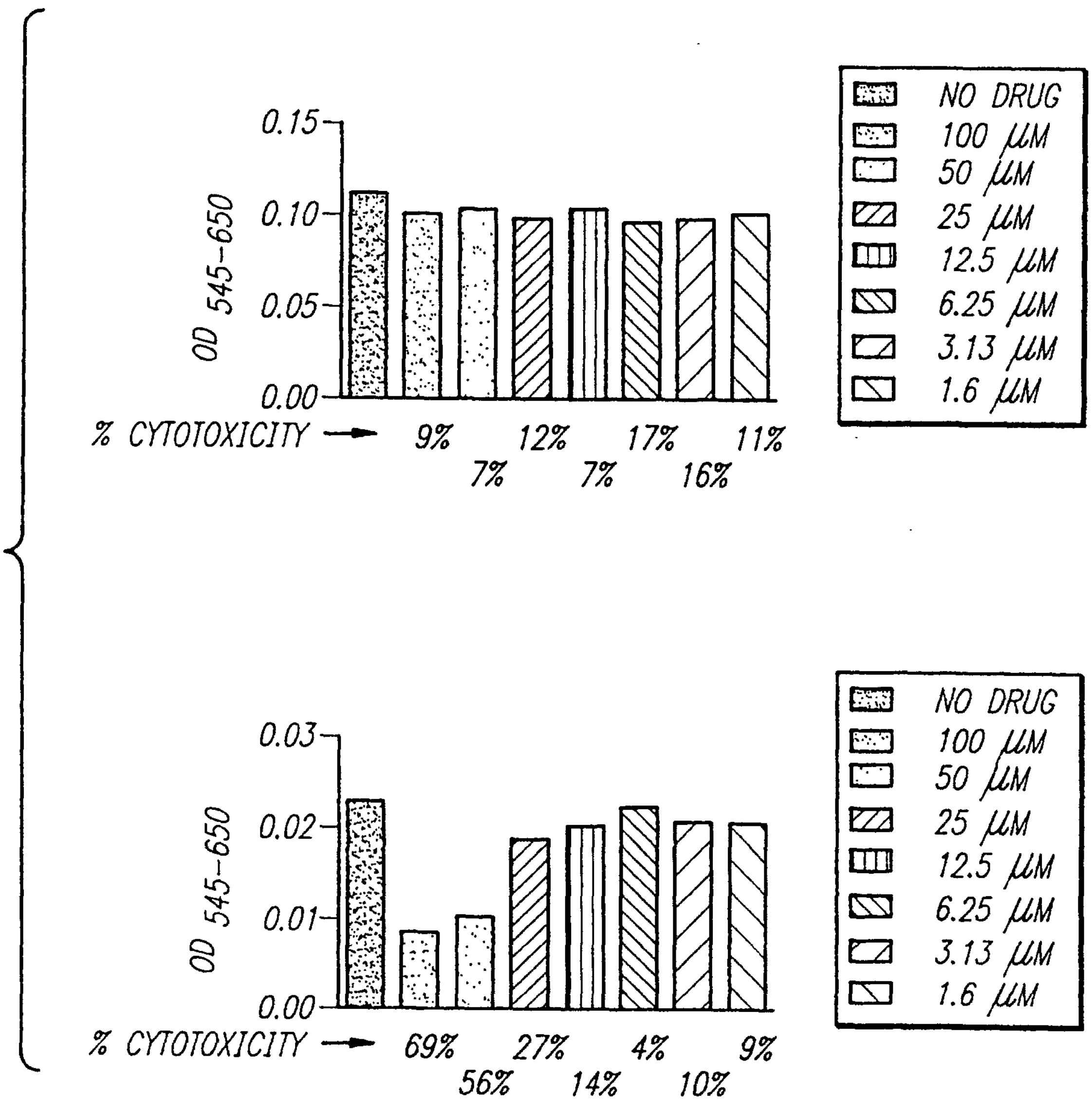
0.15
0.10
0.05
0.00
OD 545-650
% CYTOTOXICITY
9%
7%
12%
7%
17%
16%
11%
NO DRUG
100 μM
50 μM
25 μM
12.5 μM
6.25 μM
3.13 μM
1.6 μM
0.03
0.02
0.01
0.00
OD 545-650
% CYTOTOXICITY
69%
56%
27%
14%
4%
10%
9%
NO DRUG
100 μM
50 μM
25 μM
12.5 μM
6.25 μM
3.13 μM
1.6 μM

FIG. 12A

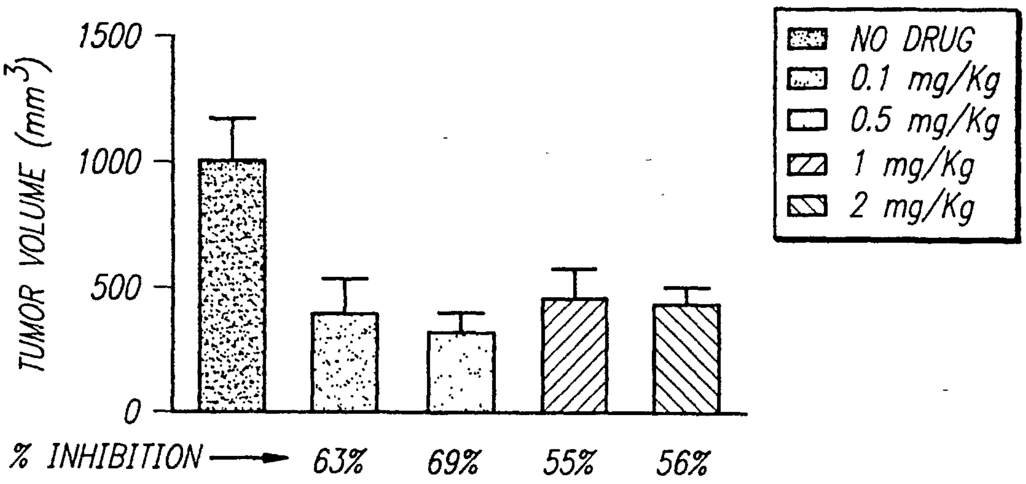


FIG. 12B

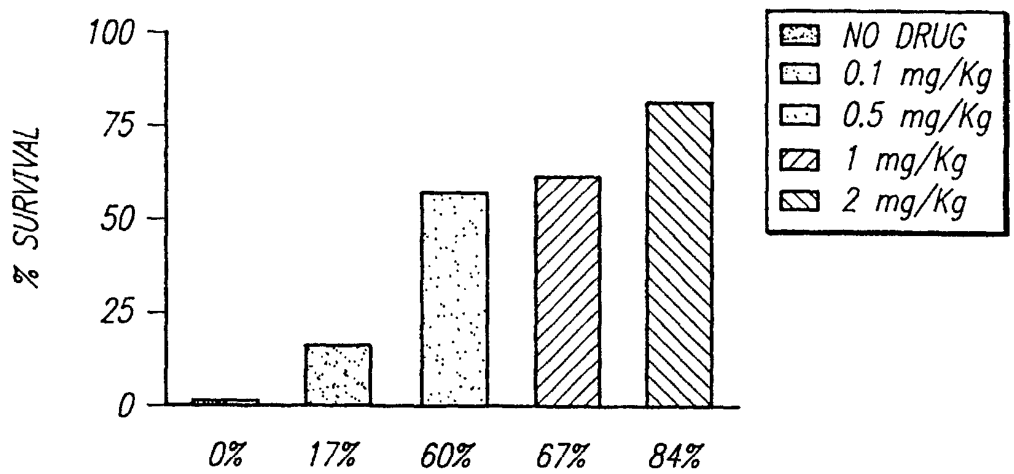

FIG. 13

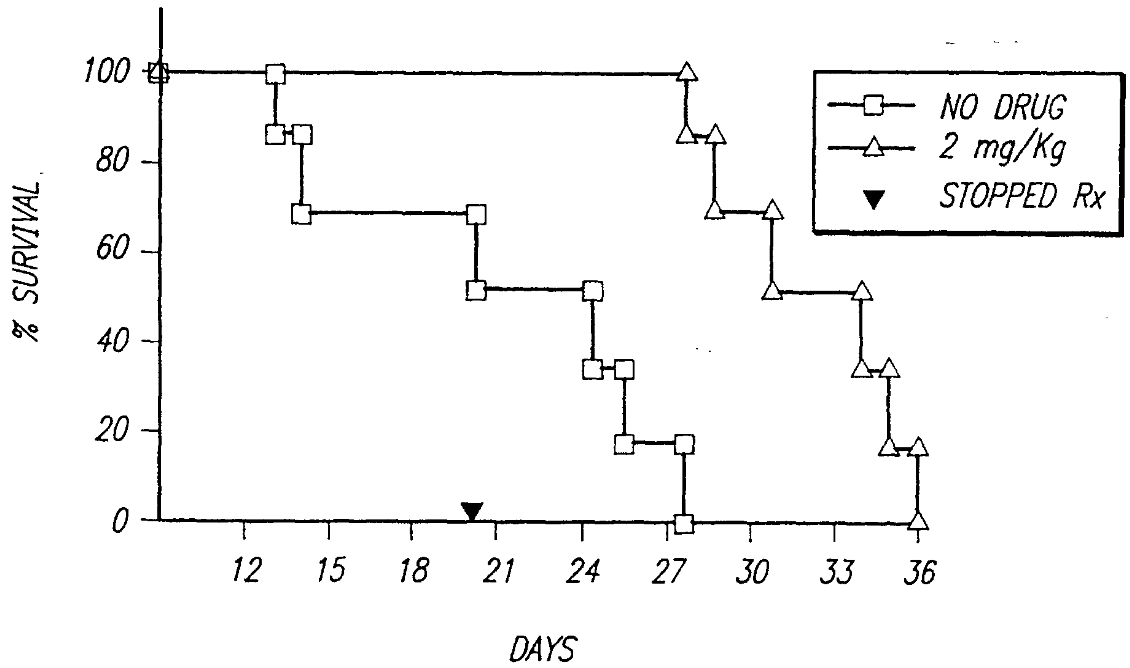
% SURVIVAL
100
80
60
40
20
0
12
15
18
21
24
27
30
33
36
DAYS
NO DRUG
2 mg/Kg
STOPPED Rx

FIG. 14

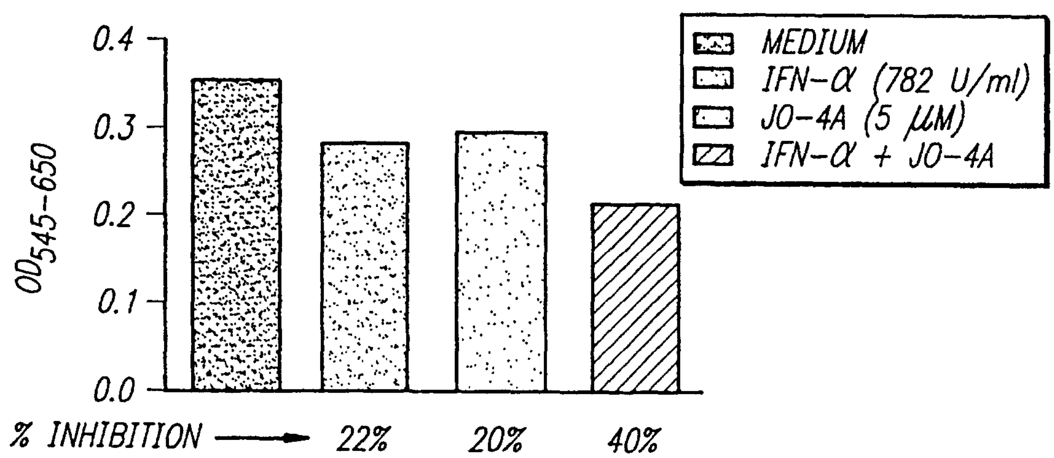
OD545-650
0.4
0.3
0.2
0.1
0.0
% INHIBITION
22%
20%
40%
MEDIUM
IFN-α (782 U/ml)
JO-4A (5 μM)
IFN-α + JO-4A

FIG. 15A

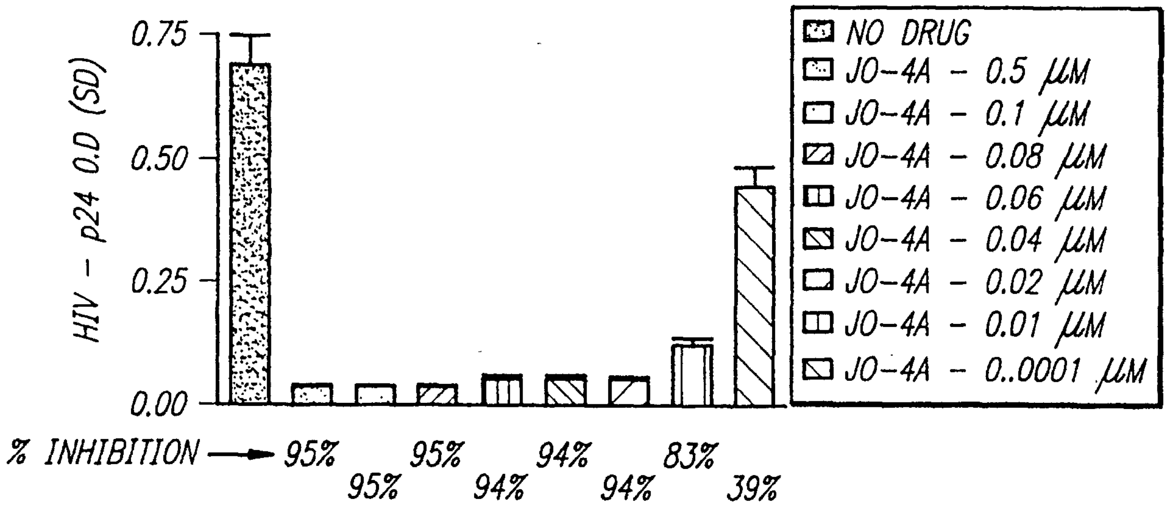


FIG. 15B

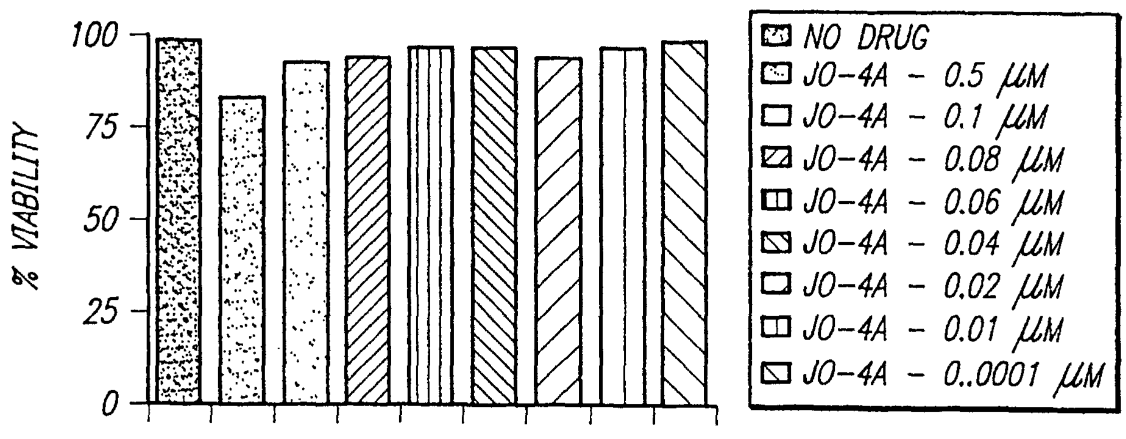

FIG. 16A

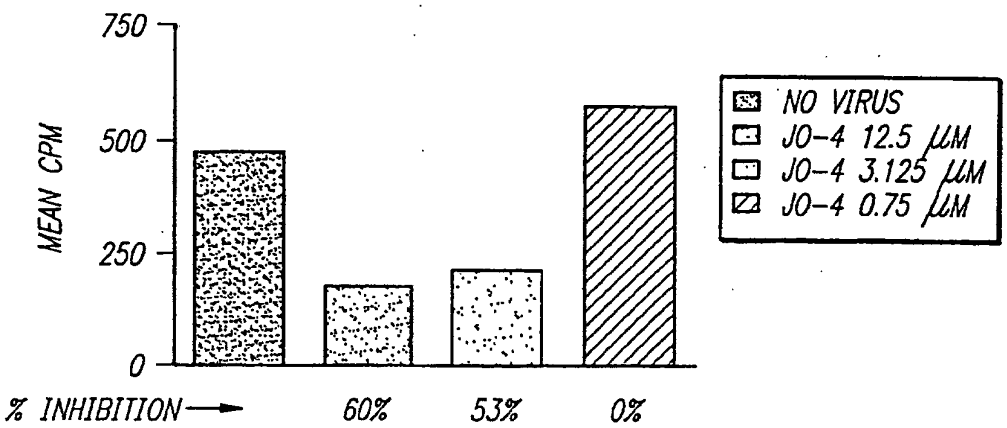


FIG. 16B

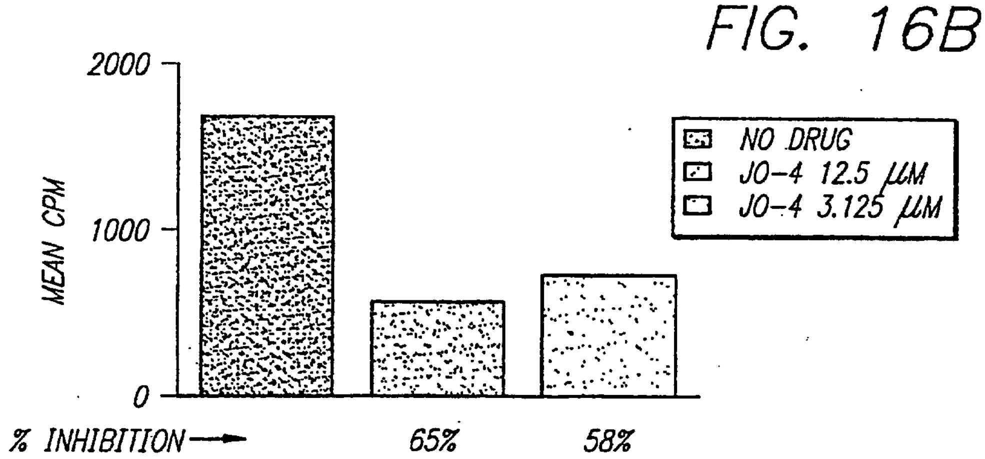

FIG. 17A

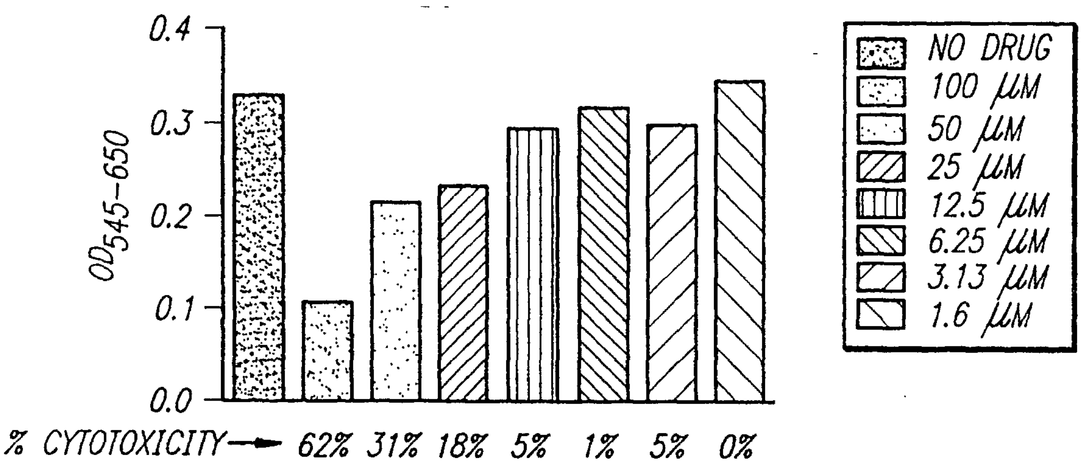


FIG. 17B